# FASCICULE DE BREVET EUROPEEN

(11) **EP 3 131 733 B1**
(45) Date de publication et mention de la délivrance du brevet: **06.06.2018**
(21) Numéro de dépôt: 15713896.7
(22) Date de dépôt: 02.04.2015
(51) Int. Cl.: B29C 49/46, B29C 49/42, B29C 49/06, B29C 49/36, A61L 2/20, A61L 2/18, A61L 2/22, A61L 2/24, B65B 43/50, B65B 65/02, B65B 3/02, B65B 7/16, B29L 31/00

(54) **INSTALLATION POUR LA PRODUCTION DE RÉCIPIENTS COMPORTANT UN DISPOSITIF DE DÉSINFECTION D'UNE ROUE DE TRANSFERT**
ANLAGE ZUR HERSTELLUNG VON BEHÄLTERN, MIT EINER VORRICHTUNG ZUR DESINFEKTION EINES TRANSFERRADS
FACILITY FOR PRODUCING CONTAINERS, COMPRISING A DEVICE FOR DISINFECTING A TRANSFER WHEEL

(30) Priorité: 17.04.2014 FR 1453491
(43) Date de publication de la demande: 22.02.2017
(73) Titulaire: Sidel Participations, 76930 Octeville-sur-Mer (FR)
(72) Inventeur: MONDOLO, Christopphe, F-76930 Octeville sur Mer (FR); DEMARE, Jérôme, F-76930 Octeville sur Mer (FR); LETELLIER, Sandy, F-76930 Octeville sur Mer (FR)
(74) Mandataire: Grassin d'Alphonse, Emmanuel Jean Marie
(86) Numéro de dépôt international: PCT/EP2015/057410
(87) Numéro de publication internationale: WO 2015/158563

(56) Documents cités:
- DE-A1-102011 122 853
- US-A1- 2009 081 326
- US-A1- 2011 250 307
- US-B2- 7 938 639
- US-B2- 8 083 512

## Description

La présente invention concerne une installation pour la production de récipients comportant un dispositif de désinfection d'une roue de transfert.

La présente invention concerne plus particulièrement une installation pour la production de récipients, notamment de bouteilles, comportant au moins :
- une première enceinte de protection délimitant une première zone à l'intérieur de laquelle est agencée au moins une unité de soufflage de récipients,
- une deuxième enceinte de confinement qui, au moins en partie accolée à la première enceinte attenante par une partie commune, délimite une deuxième zone stérile à l'intérieur de laquelle est agencée au moins une unité de remplissage des récipients fabriqués,
- au moins une ouverture qui, réalisée dans ladite partie commune desdites première et deuxième enceintes, est destinée à permettre le transfert des récipients de l'unité de soufflage à l'unité de remplissage,
- des moyens d'obturation de ladite ouverture qui sont aptes à être déplacés sélectivement entre une position ouverte dans laquelle les moyens d'obturation autorisent ledit transfert des récipients à travers l'ouverture et une position fermée dans laquelle les moyens d'obturation interdisent tout transfert en isolant la deuxième enceinte pour réaliser une décontamination de l'unité de remplissage,
- un dispositif de transfert comportant au moins une roue de transfert qui est adjacente à ladite ouverture pour transférer les récipients entre l'unité de soufflage et l'unité de remplissage, ladite roue de transfert comportant au moins une partie supérieure de transfert des récipients qui est montée mobile entre au moins une première position de transfert occupée lorsque l'installation est dans un mode de production et une deuxième position escamotée occupée lorsque l'installation est dans un mode de décontamination, le déplacement de ladite partie supérieure de la roue montée mobile entre lesdites positions de transfert et escamotée étant commandé depuis l'extérieur de l'installation, et
- un système d'entraînement de la roue de transfert pour entraîner en rotation au moins la partie supérieure de transfert des récipients de la roue lorsque, en mode de production, ladite partie supérieure occupe ladite position de transfert.

Le document WO-2010/081759 décrit et représente un exemple d'une telle installation pour la production de récipients, notamment de bouteilles.

L'installation de production est utilisée pour la fabrication de récipients en matière plastique, en particulier en Polyéthylène téréphtalate (PET).

Les récipients sont généralement fabriqués en procédant successivement au conditionnement thermique de préformes en matière plastique dans un four, puis à la transformation des préformes en bouteilles dans une unité de soufflage, la mise en forme étant par exemple réalisée par soufflage ou par étirage-soufflage dans un moule, et enfin au transfert des récipients obtenus vers une unité de remplissage et de bouchage.

L'installation comporte ainsi, en amont de l'unité de remplissage, une unité de fabrication comportant de préférence un four de conditionnement thermique et une unité de soufflage.

Généralement, l'unité de remplissage et l'unité de fabrication, à tout le moins l'unité de soufflage, sont juxtaposées pour obtenir une installation de production qui soit compacte et réalise intégralement le processus de production des récipients, jusqu'à l'obtention de produits finis.

Tel qu'expliqué dans le document WO-2010/081759, on recherche dans une telle installation de production à réduire par tous moyens les risques de contamination des récipients, lesquels récipients sont de surcroît susceptibles d'être remplis avec des produits plus ou moins sensibles à de tels risques.

Par conséquent, il est connu d'y mettre en oeuvre différentes actions aux seules fins de contrôler et de maîtriser la qualité microbiologique de l'environnement de production, en particulier l'élimination des agents pathogènes, tels que les germes, spores, bactéries, etc. qui sont susceptibles d'affecter le produit contenu dans les récipients en le rendant notamment impropre à la consommation.

Pour ce faire, les actions ne visent pas exclusivement la décontamination des récipients mais également celles des préformes à partir desquelles ils sont fabriqués ainsi que celle de l'installation elle-même en général.

Les documents suivant de l'état de la technique : WO-2006/136498 ; WO-2008/049876 ; FR-2.915.127, constituent des exemples non limitatifs illustrant de telles actions et on se reportera avantageusement pour de plus amples détails à chacun de ces documents par ailleurs cités en préambule du document WO-2010/081759.

Bien entendu, ces différents exemples d'actions sont susceptibles d'être simultanément mis en oeuvre dans une même installation pour réduire de manière drastique les risques de contamination.

Dans le processus de production, l'opération de remplissage du récipient est usuellement reconnue comme étant la plus sensible au regard des risques de contamination.

Les récipients introduits dans l'unité de remplissage ne sont toutefois que l'un des principaux vecteurs de contamination.

En effet, des agents pathogènes dès lors qu'ils sont présents dans l'environnement direct des récipients, depuis l'air jusqu'aux organes des unités de l'installation, sont notamment susceptibles de contaminer le volume intérieur du récipient.

C'est la raison pour laquelle, outre les traitements de stérilisation ou d'aseptisation visant directement le produit destiné à être introduit dans le récipient et le récipient lui-même, on procède également à une décontamination de l'unité de remplissage par voie chimique, en particulier par pulvérisation de solutions stérilisantes comme de l'hydroxyde de sodium (NaOH) ou du peroxyde d'hydrogène (H₂O₂).

La décontamination de l'unité de remplissage correspond à un mode de fonctionnement, dit de décontamination, de l'installation qui précède et/ou succède à la mise en oeuvre du mode de fonctionnement, dit de production, de l'installation dans lequel des récipients sont fabriqués.

Pour pouvoir procéder à la mise en oeuvre du mode de décontamination de l'unité de remplissage, il est nécessaire d'isoler l'unité de remplissage du reste de l'installation et tout particulièrement de l'unité de soufflage adjacente.

En effet, si les organes de l'unité de remplissage sont réalisés dans des matériaux appropriés, tels que de l'inox 316L, pour résister à l'agression chimique des solutions stérilisantes précitées qui sont utilisées pour la décontamination, cela n'est toutefois pas le cas de l'unité de soufflage, ni généralement du dispositif de transfert attenant interposé entre ces unités.

Ainsi, les solutions stérilisantes utilisées pour la décontamination sont susceptibles de provoquer des agressions chimiques indésirables, notamment la corrosion d'organes de l'unité de soufflage, tels que les moules, voir également d'organes du dispositif de transfert.

Le document WO-2010/081759 décrit la mise en oeuvre de la décontamination de l'unité de remplissage et décrit tout particulièrement les risques de contamination liés à l'intervention d'un opérateur pénétrant dans la première enceinte pour y effectuer des opérations de démontage et/ou remontage d'au moins une partie de la roue de transfert.

De telles opérations de démontage et/ou remontage d'au moins une partie de la roue de transfert des récipients sont requises pour permettre la fermeture par des moyens d'obturation de l'ouverture de communication à travers laquelle s'étend ladite partie de la roue pour assurer le transfert des récipients en mode de production.

Dans l'installation selon le document WO-2010/081759, ladite ouverture de communication est ménagée dans une partie commune entre la première enceinte comportant l'unité de soufflage et la deuxième enceinte comportant l'unité de remplissage, lesdits moyens d'obturation permettant d'isoler sélectivement l'unité de remplissage lors de la décontamination.

Tel qu'expliqué dans ce document, l'opérateur constitue un vecteur potentiel important d'introduction d'agents pathogènes à l'intérieur de l'installation induisant des risques de contamination de différents ordres et multiples.

Pour y remédier, le document WO-2010/081759 propose une nouvelle conception d'installation et de la roue de transfert selon laquelle l'escamotage de la partie supérieure de transfert des récipients de la roue, qui s'étend à travers l'ouverture en mode de production, est susceptible d'être réalisée par un opérateur depuis l'extérieur de l'installation.

Grâce à cette nouvelle conception, l'opérateur demeure à l'extérieur de l'installation, sans jamais pénétrer physiquement dans la première enceinte pour réaliser, manuellement ou automatiquement, le déplacement d'au moins la partie supérieure de transfert des récipients de la roue entre une position de transfert et une position escamotée.

Avantageusement, l'opérateur intervenant à distance ne peut à aucun moment former un vecteur d'introduction d'agents pathogènes à l'intérieur du volume de l'installation délimité par les enceintes, de sorte que les risques de contamination précités sont supprimés.

De plus, une telle conception permet également de remplacer les opérations de démontage et de remontage effectuées auparavant par des opérations plus simples et rapides permettant d'obtenir l'escamotage d'au moins la partie supérieure de transfert des récipients de la roue et la fermeture de l'ouverture par les moyens d'obturation afin de décontaminer l'unité de remplissage.

La mise en oeuvre de la décontamination par voie chimique de l'unité de remplissage avec la nouvelle conception de roue de transfert décrite dans le document WO-2010/081759 est un exemple d'action améliorant encore la sécurité alimentaire et répondant ainsi à la demande d'un contrôle et d'une maîtrise toujours plus grande de la qualité microbiologique de l'environnement de production des récipients, en particulier de l'élimination des agents pathogènes, tels que les germes, spores, bactéries, etc.

Selon le document FR-2.915.127 précité, il est connu d'équiper l'installation d'un système d'insufflation d'air filtré à l'intérieur des enceintes pour y établir une surpression propre à limiter les risques de contamination des préformes dans la zone de sortie du four, comme des récipients fabriqués.

Toutefois, si un tel système d'insufflation d'air filtré prévient l'introduction d'agents pathogènes dans l'installation, il n'a pas d'action sur les agents pathogènes pouvant être présents dans la première zone que délimite la première enceinte et à l'intérieur de laquelle sont agencées l'unité de soufflage et le dispositif de transfert.

L'utilisation de solutions stérilisantes comme l'hydroxyde de sodium (NaOH) ou le peroxyde d'hydrogène (H₂O₂) pour réaliser une décontamination dans la première zone se heurte notamment aux risques de corrosion d'organes réalisés dans des matériaux autres que l'inox, en particulier pour les moules de l'unité de soufflage.

En conséquence, le dispositif de transfert agencé à l'intérieur de la première enceinte avec l'unité de soufflage ne fait parallèlement pas non plus l'objet d'action particulière visant à détruire les agents pathogènes pouvant être présents sur au moins une roue de transfert des récipients.

Or, en mode de production, les récipients sont en contact avec tant avec les moyens de maintien qu'avec les moyens de guidage associés d'une telle roue de transfert de sorte qu'une contamination des récipients est alors susceptible de se produire.

La contamination d'un récipient en cours de transfert est un risque particulièrement critique car un récipient contaminé transféré vers l'unité de remplissage constitue alors un vecteur d'introduction d'agents pathogènes.

De plus et indépendamment des récipients, de tels agents pathogènes présents sur une roue de transfert sont également susceptibles d'être aéroportés jusqu'à la deuxième zone dans laquelle est agencée l'unité de remplissage.

Ces risques de contamination associés à la roue de transfert sont susceptibles d'affecter l'ensemble des différentes actions mises en oeuvre pour éliminer les agents pathogènes, tel que par exemple la décontamination de l'unité de remplissage.

Le but de la présente invention est notamment de proposer une installation pour la production de récipients dans laquelle les risques de contamination des récipients soient encore plus réduits voire supprimés et tout particulièrement les risques de contamination associés à la présence d'agents pathogènes au niveau du dispositif de transfert.

Dans ce but, l'invention propose une installation du type décrit précédemment, caractérisée en ce que l'installation comporte un dispositif de désinfection de la roue de transfert qui est commandé depuis l'extérieur de l'installation pour désinfecter, par pulvérisation d'au moins un agent de désinfection, au moins la partie supérieure de transfert des récipients de la roue lorsque, en mode de décontamination, ladite partie supérieure de transfert des récipients de la roue occupe la position escamotée.

Grâce au dispositif de désinfection selon l'invention, ladite au moins une roue de transfert est désinfectée, éliminant les agents pathogènes qui pourraient y être présents.

Avantageusement, une telle opération de désinfection de la roue de transfert est réalisée pendant que l'installation fonctionne en mode de décontamination.

De préférence, l'opération de désinfection est réalisée avant toute mise en production de récipients afin de réduire voir supprimer les risques de contamination ultérieure de l'unité de remplissage, notamment de contamination d'un récipient par contact entre le récipient et la roue de transfert en cours de production.

Selon d'autres caractéristiques de l'invention :
- le dispositif de désinfection est constitué par au moins un pistolet agencé à l'intérieur de l'installation et apte à être manipulé par un opérateur depuis l'extérieur de l'installation, par l'intermédiaire de moyens de manipulation à distance, pour pulvériser ledit au moins un agent de désinfection sur au moins ladite partie supérieure de transfert des récipients de la roue ;
- le dispositif de désinfection est constitué par au moins un module de désinfection automatique qui, commandé depuis l'extérieur de l'installation, comporte des moyens de pulvérisation dudit au moins un agent de désinfection pour désinfecter au moins ladite partie supérieure de transfert des récipients de la roue ;
- ledit module de désinfection automatique est monté mobile entre au moins :
   - une position de désinfection, occupée en mode de décontamination, dans laquelle lesdits moyens de pulvérisation sont aptes à désinfecter au moins la partie supérieure de transfert des récipients de la roue ; et
   - une position de repos, occupée en mode de production, dans laquelle ledit module de désinfection est escamoté ;
- le module de désinfection automatique comporte au moins un bras desdits moyens de pulvérisation de l'agent de désinfection et ledit bras est déplacé sélectivement par au moins un actionneur pour être déployé vers la position de désinfection ou escamoté vers la position de repos ;
- lesdits moyens de pulvérisation de l'agent de désinfection sont montés mobiles en rotation autour d'un axe, respectivement entre au moins une première position angulaire et une deuxième position angulaire, pour réaliser dans ladite position de désinfection un balayage d'au moins ladite partie supérieure de transfert des récipients de la roue ;
- ladite partie supérieure de transfert des récipients de la roue comporte au moins des moyens de maintien auxquels sont associés des moyens de guidage qui, en mode de production, participent respectivement au transfert des récipients et le module de désinfection automatique comporte au moins des premiers moyens de pulvérisation pour désinfecter lesdits moyens de maintien et des deuxièmes moyens de pulvérisation pour désinfecter lesdits moyens de guidage ;
- ledit au moins un agent de désinfection est formé en tout ou en partie par un composé de la famille des alcools qui est pulvérisé à l'état liquide par le dispositif de désinfection pour désinfecter au moins ladite partie supérieure de transfert des récipients de la roue ;
- au moins une partie du dispositif de désinfection ou la partie supérieure de transfert des récipients de la roue occupant ladite position escamotée est apte à effectuer un mouvement relatif par rapport à l'autre pour parfaire l'application dudit au moins un agent de désinfection ;
- ledit système d'entraînement de la roue de transfert est apte à entraîner en rotation au moins ladite partie supérieure de transfert des récipients de la roue lorsque, en mode de décontamination, ladite partie supérieure de transfert des récipients occupe ladite position escamotée, grâce à quoi au moins ladite partie supérieure de transfert des récipients de la roue est animée d'un mouvement relatif par rapport au dispositif de désinfection ;
- ledit système d'entraînement de la roue de transfert des récipients comporte au moins un arbre primaire réalisé en au moins deux parties, respectivement une première partie supérieure à laquelle est reliée au moins ladite partie supérieure de transfert des récipients et une deuxième partie inférieure d'entraînement, lesdites première et deuxième parties étant liées en rotation à un arbre secondaire ;
- les première et deuxième parties de l'arbre primaire sont respectivement liées en rotation à l'arbre secondaire par coopération de formes, de préférence ladite liaison en rotation est réalisée par engrènement ;
- un mécanisme secondaire d'embrayage est associé à l'arbre secondaire pour en commander sélectivement l'entraînement par des moyens motorisés ;
- ledit mécanisme secondaire d'embrayage occupe au moins :
   - un état embrayé lorsque ladite au moins une partie supérieure de transfert des récipients de la roue est en position escamotée pour accoupler en rotation, par l'intermédiaire de l'arbre secondaire, ladite partie supérieure dudit arbre primaire auxdits moyens motorisés afin d'entraîner en rotation ladite partie supérieure de transfert des récipients, et
   - un état débrayé lorsque ladite au moins une partie supérieure de transfert des récipients de la roue est en position de transfert pour désaccoupler ledit arbre secondaire desdits moyens motorisés.

D'autres caractéristiques et avantages de l'invention apparaîtront à la lecture de la description qui va suivre pour la compréhension de laquelle on se reportera aux dessins annexés dans lesquels :
- la figure 1 est une vue de dessus qui représente partiellement un exemple de réalisation d'une installation de production de récipients et qui illustre plus particulièrement la partie commune de jonction entre les enceintes de l'unité de soufflage et de l'unité de remplissage dans laquelle est aménagée une ouverture pour le transfert des récipients ;
- la figure 2 est une vue de trois-quarts arrière qui représente l'installation selon la figure 1 et qui illustre la roue de transfert du dispositif de transfert qui, adjacente à l'ouverture, est en position de transfert ;
- les figures 3 et 4 sont des représentations schématiques du système d'entraînement de la roue de transfert, qui illustrent ledit système respectivement dans les positions de transfert et de désinfection dans lesquelles au moins la partie supérieure de transfert des récipients de la roue de transfert est apte à être entraînée en rotation ;
- les figures 5 à 7 sont des vues en perspective qui représentent en détails une roue de transfert comportant un système d'entraînement selon les figures 3 et 4 et qui illustrent la partie supérieure de la roue de transfert en position de transfert et en position escamotée ;

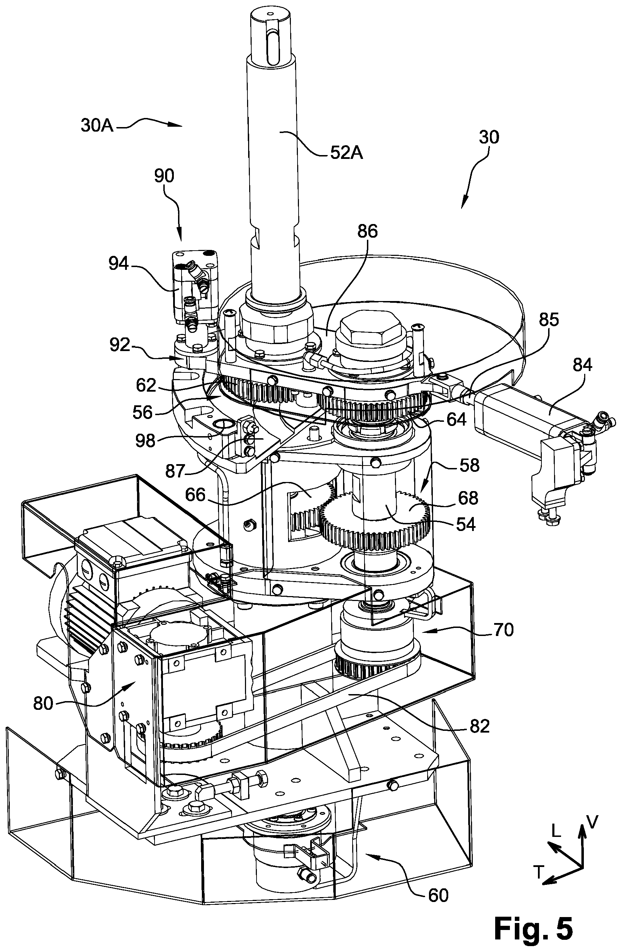

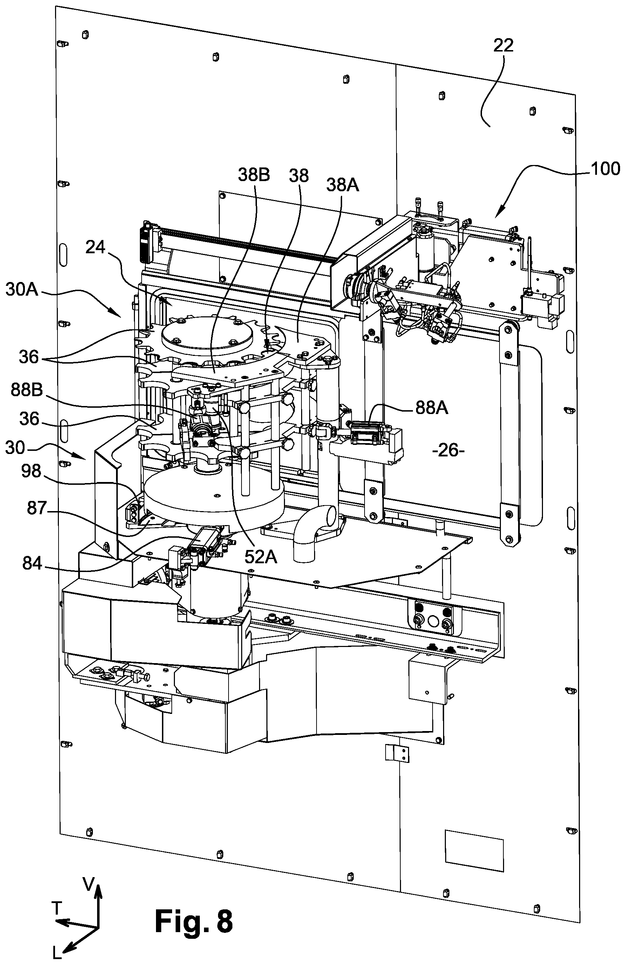

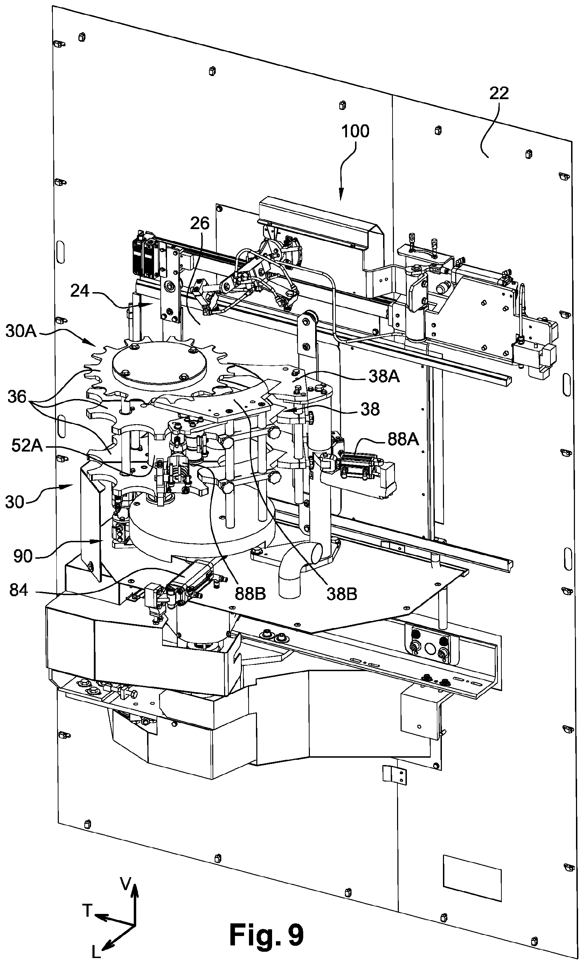

- les figures 8 et 9 sont des vues de trois-quarts en perspective qui représentent partiellement l'installation et plus particulièrement la roue de transfert selon les figures 5 à 7 adjacente à la partie commune des première et deuxième enceintes comportant l'ouverture et qui illustrent respectivement l'installation en mode de transfert et en mode de décontamination, la partie supérieure de la roue de transfert occupant respectivement la position de transfert et la position escamotée tandis que le dispositif de désinfection y occupe respectivement la position de repos et la position de désinfection ;
- la figure 10 est une vue en perspective qui représente un dispositif de désinfection selon le deuxième mode de réalisation entièrement automatisée de l'installation ;
- les figures 11 et 12 sont des vues en perspectives qui représentent le dispositif de désinfection selon la figure 10 et qui illustrent la pulvérisation de l'agent de désinfection par les buses dans la première position angulaire et dans la deuxième position angulaire.

Dans la suite de la description, des éléments similaires ou identiques seront désignés par les mêmes références.

Dans la description, on utilisera à titre non limitatif les expressions telles que "amont" et "aval", "supérieur" et "inférieur", "intérieur" et "extérieur", "avant" et "arrière" et les orientations longitudinale, verticale et transversale en référence aux trièdres (L, V, T) représentés sur les figures et aux définitions données dans la description.

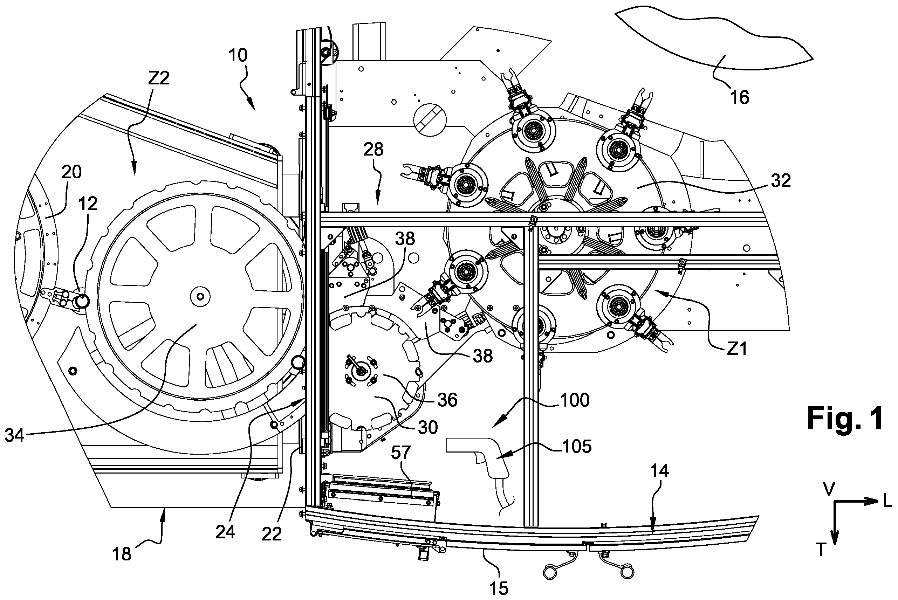

On a représenté à la figure 1, un exemple de réalisation d'une installation 10 pour la production de récipients 12, notamment, mais non exclusivement, de bouteilles.

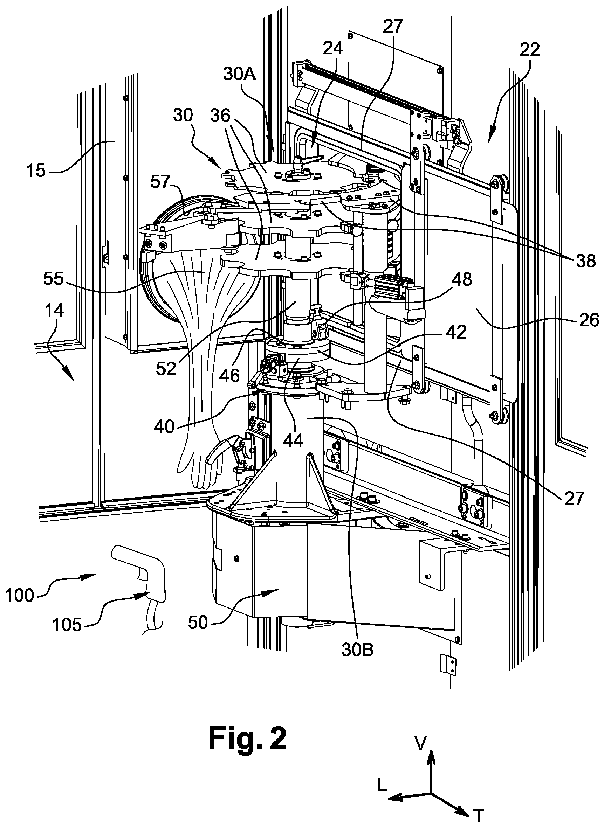

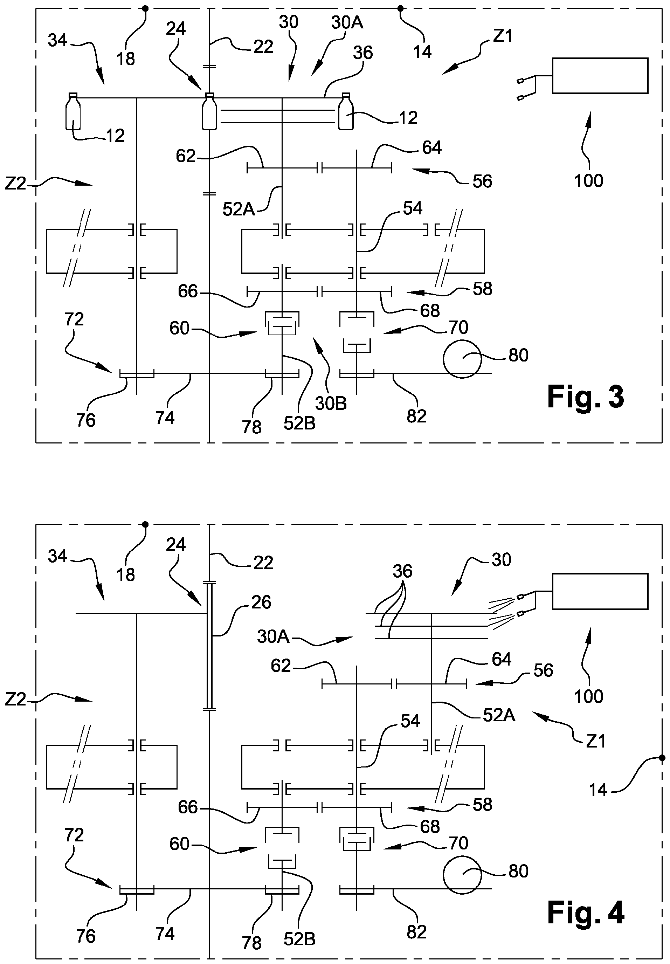

On décrira ci-après un premier mode de réalisation d'une installation 10 de production de récipients 12 qui est analogue à celle décrite dans le document WO-2010/081759 dont les figures 3 et 5 reprises correspondent aux présentes figures 1 et 2.

On se reportera donc avantageusement à ce document pour une description plus détaillée de l'ensemble de l'installation 10.

Tel que représenté partiellement sur la figure 1, l'installation 10 comporte au moins une première enceinte 14 de protection qui délimite une première zone Z1 à l'intérieur de laquelle est agencée au moins une unité 16 de soufflage de récipients 12.

De préférence, l'unité 16 de soufflage est apte à fabriquer des récipients 12 par soufflage ou par étirage-soufflage à partir de préformes (non représentées) qui sont préalablement conditionnées thermiquement dans un four (non représenté), notamment par chauffage au moyen de lampes à rayonnements infrarouges.

De manière connue, une telle unité 16 de soufflage comporte en amont un four de chauffage pour le conditionnement thermique des préformes, ainsi que des moyens de transfert (non représentés) agencés en sortie qui sont aptes à amener chacune desdites préformes chauffées dans un des moules (non représentés).

L'unité 16 de soufflage comporte un carrousel (non représenté) à la périphérie radiale duquel sont circonférentiellement agencés lesdits moules dans lesquels les préformes chauffées sont transformées en récipient 12 par soufflage ou encore par étirage-soufflage selon les applications.

L'installation 10 comporte une deuxième enceinte 18 de confinement qui délimite une deuxième zone Z2 stérile, à l'intérieur de laquelle est agencée au moins une unité 20 de remplissage des récipients 12 fabriqués par l'unité 16 de soufflage.

La deuxième enceinte 18 de confinement est au moins en partie accolée à la première enceinte 14 attenante par une partie 22 commune.

Chacune des enceintes 14 et 18 est constituée d'un ensemble de parois verticales qui, notamment assemblées entre elles pour former globalement un parallélépipède, sont respectivement fermées au-dessus et en-dessous, par exemple par une paroi formant plafond et par le sol respectivement.

De préférence, au moins une des parois verticales des enceintes 14 et 18 comporte des portes afin de permettre leur franchissement, notamment par un opérateur, et l'accès à l'intérieur de l'installation 10.

La première enceinte 14 comporte par exemple au moins deux portes 15 d'accès formant une partie de sa paroi verticale avant.

L'installation 10 comporte au moins une ouverture 24 qui est réalisée dans ladite partie 22 commune des première et deuxième enceintes 14 et 18. L'ouverture 24 visible sur la figure 2 est destinée à permettre le transfert des récipients 12 de l'unité 16 de soufflage vers l'unité 20 de remplissage.

Avantageusement, l'installation 10 comporte des moyens 26 d'obturation de ladite ouverture 24 qui sont aptes à être déplacés sélectivement entre au moins une position ouverte et une position fermée.

La position ouverte correspond à la position dans laquelle les moyens 26 d'obturation autorisent le transfert des récipients 12 à travers l'ouverture 24 et est occupée lorsque l'installation 10 est dans un mode de fonctionnement, dit mode de production.

La position fermée correspond à la position dans laquelle les moyens 26 d'obturation interdisent tout transfert en isolant la deuxième enceinte 18.

La position fermée est en particulier destinée à permettre de procéder au moins dans la deuxième enceinte 18 à des opérations de décontamination de l'unité 20 de remplissage, lorsque l'installation 10 est dans un autre mode de fonctionnement, dit mode de décontamination.

De préférence, l'installation 10 comporte des moyens d'actionnement destinés à commander sélectivement le déplacement des moyens 26 d'obturation de l'ouverture 24 pour faire coulisser lesdits moyens 26 d'obturation entre la position ouverte correspondant au mode de production et la position fermée correspondant au mode de décontamination.

Avantageusement, les moyens 26 d'obturation assurent dans ladite position fermée une fermeture hermétique de l'ouverture 24 propre à isoler la deuxième enceinte 18 comportant l'unité 20 de remplissage de la première enceinte 14 comportant l'unité 16 de soufflage des récipients 12.

Les moyens 26 d'obturation de l'ouverture 24 sont constitués par un volet qui est monté coulissant entre lesdites positions ouverte et fermée par l'intermédiaire de deux glissières 27, respectivement une glissière supérieure et une glissière inférieure.

Les glissières 27 du volet 26 sont solidaires de la partie 22 commune et sont agencées respectivement au-dessus et au-dessous de l'ouverture 24 qui présente ici une forme globalement rectangulaire plus particulièrement visible sur la figure 2.

Les opérations de décontamination de l'unité 20 de remplissage sont réalisées par voie chimique en pulvérisant des solutions stérilisantes, telles que de l'hydroxyde de sodium (NaOH) ou du peroxyde d'hydrogène (H₂O₂), à l'intérieur de la deuxième enceinte 18.

La pulvérisation de telles solutions stérilisantes pour décontaminer la deuxième enceinte 18 est notamment rendue possible par l'utilisation d'inox ou autres matériaux compatibles pour réaliser les parties telles que celles de l'unité 20 de remplissage.

L'installation 10 comporte un dispositif 28 de transfert pour assurer le transfert des récipients 12 entre l'unité 16 de soufflage et l'unité 20 de remplissage.

Le dispositif 28 de transfert comporte au moins une roue 30 de transfert qui est adjacente à l'ouverture 24 aménagée dans la partie 22 commune et qui est destinée à transférer, en mode de production, les récipients 12 à travers ladite ouverture 24.

De préférence, le dispositif 28 de transfert comporte en plus de la roue 30 de transfert, deux autres roues 32 et 34 de transfert respectivement agencées en amont et en aval de la roue 30.

Les différentes roues 30, 32 et 34 du dispositif 28 de transfert sont agencées les unes par rapport aux autres de manière à présenter chacune une portion en arc de cercle tangente à la portion de la roue adjacente afin de déterminer une zone dans laquelle s'opère le transfert des récipients 12 de l'une à l'autre.

La première roue 32 de transfert est agencée entre l'entrée de la roue 30 de transfert et une zone de sortie du carrousel de l'unité 16 de soufflage dans laquelle les moules sont commandés en ouverture pour permettre l'extraction des récipients 12 fabriqués grâce aux moyens de soufflage ou d'étirage-soufflage associés.

La première roue 32 de transfert est destinée à assurer l'extraction des récipients 12 fabriqués hors des moules et leur transfert vers l'aval, par exemple par l'intermédiaire de bras de transfert munis à leur extrémité libre de moyens de préhension, tels que des pinces.

La deuxième roue 34 de transfert est agencée, en aval, entre la sortie de la roue 30 de transfert et un autre carrousel que comporte l'unité 20 de remplissage, les postes de remplissage des récipients 12 étant répartis circonférentiellement de manière régulière autour dudit carrousel.

Avantageusement, l'installation 10 est conçue de manière à réduire ou supprimer les différents risques de contamination, en particulier et selon les enseignements délivrés par le document WO-2010/081759 ceux liés à l'intervention d'un opérateur pour intervenir sur la roue 30 de transfert en vue de permettre l'ouverture ou la fermeture de l'ouverture 24.

La roue 30 de transfert comporte au moins une partie qui est respectivement montée mobile entre au moins une première position de transfert et une deuxième position escamotée.

La première position de transfert est occupée lorsque l'installation 10 est dans le mode de production et la deuxième position escamotée lorsque l'installation 10 est dans le mode de décontamination.

De préférence, la roue 30 de transfert comporte une partie 30A supérieure de transfert des récipients et une partie 30B inférieure d'entraînement de la roue 30.

La partie 30A supérieure comporte au moins des moyens 36 de maintien qui s'étendent en partie à travers l'ouverture 24 et, en mode de production, coopèrent avec les récipients 12 pour les transférer.

Les moyens 36 de maintien sont par exemple constitués par trois plateaux superposés qui sont pourvus chacun circonférentiellement d'encoches, chaque encoche étant destinée à coopérer avec une partie du récipient.

Le plateau supérieur coopère avec le col du récipient, le supportant généralement par l'intermédiaire d'une collerette radiale dont le récipient est pourvu, tandis que les plateaux intermédiaire et inférieur coopèrent avec le corps du récipient, le bloquant latéralement pour le maintenir vertical lors du transfert.

Avantageusement, la roue 30 de transfert comporte des moyens 38 de guidage qui, associés aux moyens 36 de maintien, interviennent pour assurer le transfert des récipients 12.

Tel que décrit dans le document WO-2010/081759, ladite au moins une partie mobile de la roue 30 de transfert est de préférence constituée par la partie supérieure 30A de transfert des récipients tandis que la partie 30B inférieure d'entraînement de la roue 30 est une partie fixe.

La partie 30A supérieure de la roue 30 de transfert est montée mobile entre au moins :
- la première position de transfert dans laquelle au moins ladite partie 30A supérieure de la roue 30 s'étend à travers l'ouverture 24 pour assurer le transfert des récipients 12 lors du fonctionnement en mode de production de l'installation 10, et
- la deuxième position escamotée dans laquelle au moins ladite partie supérieure 30A de la roue 30 est déplacée pour permettre la fermeture de l'ouverture 24 par les moyens 26 d'obturation associés en vue du fonctionnement de l'installation 10 en mode de décontamination.

De préférence, la roue 30 de transfert de l'installation 10 est dans ce premier mode de réalisation d'une conception analogue à celle décrite dans le document WO-2010/081759.

La roue 30 de transfert comporte au moins une bride 40 comportant un élément 42 supérieur et un élément 44 inférieur qui sont respectivement solidaires de la partie 30A supérieure et de la partie 30B inférieure.

Des moyens 46 d'articulation, tels qu'un pivot, sont agencés entre l'élément 42 supérieur et l'élément 44 inférieur de la bride 40 pour permettre le déplacement de la partie 30A supérieure par rapport à la partie 30B inférieure.

De préférence, la roue 30 de transfert comporte des moyens 48 de verrouillage aptes à immobiliser la partie 30A supérieure mobile de la roue 30 dans au moins l'une desdites positions de transfert ou escamotée.

L'installation 10 comporte un système 50 d'entraînement de la roue 30 de transfert pour assurer l'entraînement en rotation d'au moins la partie 30A supérieure de transfert des récipients 12 de la roue 30 lorsque, en mode de production, ladite partie 30A supérieure occupe la position de transfert.

Dans le document WO-2010/081759, ledit système 50 d'entraînement de la roue 30 de transfert comporte un arbre d'entraînement comportant ladite bride 40 disposée à la jonction d'un premier tronçon d'arbre solidaire de l'élément 42 supérieur de bride et un deuxième tronçon d'arbre solidaire de l'élément 44 inférieur de bride.

Les moyens 36 de maintien sont notamment montés sur le premier tronçon d'arbre pour former ladite partie 30A supérieure de transfert des récipients de la roue 30.

Le deuxième tronçon d'arbre est relié à des moyens motorisés qui sont avantageusement constitués par les moyens d'entraînement en rotation de l'unité 20 de remplissage et/ou de la deuxième roue 34 de transfert afin d'assurer, en mode de production, un entraînement synchrone suivant le sens de transfert des récipients 12, depuis l'unité 16 de soufflage en amont vers l'unité 20 de remplissage en aval.

Les moyens d'entraînement en rotation de l'unité 20 de remplissage comportent au moins des moyens motorisés et des moyens de transmission, tels qu'une courroie, qui assurent la transmission du couple entre lesdits moyens motorisés et l'arbre de la roue 30 de transfert.

Le système 50 d'entraînement comporte un mécanisme d'embrayage qui est associé à l'arbre d'entraînement pour accoupler sélectivement en rotation ledit arbre et l'entraîner en rotation uniquement en mode de production lorsque la partie 30A supérieure occupe sa position de transfert.

Lorsque la partie 30A supérieure occupe sa position escamotée en mode de décontamination, le mécanisme d'embrayage est alors ouvert pour interrompre l'entraînement en rotation de la partie 30A supérieure.

Selon les enseignements du document WO-2010/081759, le déplacement de la partie 30A supérieure de la roue 30 de transfert entre lesdites positions de transfert et escamotée est réalisé, manuellement ou automatiquement, depuis l'extérieur de l'installation 10, c'est à dire depuis l'extérieur de la première enceinte 14 à l'intérieur de laquelle est agencée ladite roue 30 de transfert.

En variante, l'ensemble de la roue de transfert 30 est montée mobile, par exemple en étant montée sur un chariot apte à permettre son déplacement entre lesdites positions de transfert et escamotée et toujours depuis l'extérieur de l'installation.

Dans une telle variante la roue de transfert 30 comporte avantageusement ses propres moyens d'entraînement en rotation, indépendants de ceux de l'unité 20 de remplissage.

Avantageusement, la présence physique d'un opérateur à l'intérieur de l'installation 10 et tout particulièrement dans la première zone Z1 de la première enceinte 14 n'est donc plus nécessaire, grâce à quoi les risques de contamination associés sont totalement supprimés.

Tel qu'expliqué en préambule, le but de la présente invention est de réduire les risques microbiologiques au sein d'une installation 10 de production de récipients, en luttant contre les différents risques de contamination des récipients par des agents pathogènes et cela pour améliorer encore et toujours la sécurité alimentaire des récipients.

Dans ce but, l'invention propose de réaliser une opération de désinfection d'au moins une partie de la roue 30 de transfert de l'installation 10 de production.

Avantageusement, l'opération de désinfection est réalisée lorsque l'installation 10 est en mode de décontamination, par exemple parallèlement à la mise en oeuvre de l'opération de décontamination de l'unité 20 de remplissage.

Avantageusement, la désinfection de la roue 30 de transfert s'opère en temps masqué, sans affecter la production des récipients qui succède ou précède la mise en oeuvre desdites opérations de décontamination de l'unité 20 de remplissage et de désinfection d'au moins une partie de la roue 30 de transfert.

L'emploi du terme de « désinfection » est notamment destiné à faciliter la distinction entre la nouvelle opération mise en oeuvre pour traiter la roue 30 de transfert et celle de « décontamination » réalisée pour traiter l'unité 20 de remplissage.

On comprendra cependant que la désinfection comme la décontamination poursuivent un seul et même but, c'est à dire la destruction des agents pathogènes (germes, spores, bactéries, etc.) susceptibles d'être présents sur les surfaces traitées, notamment mais non exclusivement, des surfaces destinées à être en contact avec les récipients.

La quantité et le type d'agents pathogènes détruits sont notamment fonction des produits utilisés lors desdites opérations de décontamination et de désinfection.

On rappelle que la quantité d'agents pathogènes est susceptible d'être dénombrée par comptage après notamment des opérations de lavage, de filtration et de mise en culture.

On détermine ainsi une réduction logarithmique du nombre d'agents pathogènes par exemple dite de l'ordre de 3Log (ou encore 3D) équivalent à 1000 unités (10³).

A titre d'exemple, l'opération de décontamination par voie chimique de l'unité 20 de remplissage avec des solutions stérilisantes comme l'hydroxyde de sodium (NaOH) ou le peroxyde d'hydrogène (H₂O₂) permettent d'obtenir des résultats pouvant aller jusqu'à 6Log.

De telles solutions stérilisantes sont également susceptibles d'être utilisées pour l'opération de désinfection d'au moins une partie de la roue de transfert, notamment lorsque les surfaces traitées sont réalisées dans des matériaux appropriés, tels que de l'inox.

Toutefois, indépendamment des risques de corrosion liés à l'utilisation de solutions stérilisantes comme l'hydroxyde de sodium (NaOH) ou le peroxyde d'hydrogène (H₂O₂), il convient également de prendre en considération l'élimination ultérieure desdites solutions stérilisantes à la fin de l'opération de désinfection.

En effet, la réglementation dans le domaine agro-alimentaire impose généralement des exigences quant à la présence résiduelle de certains produits, tels que la présence de traces de solution stérilisante, dans ou sur les récipients.

Avantageusement, l'opération de désinfection selon l'invention est réalisée avec au moins un agent de désinfection qui est formé en tout ou en partie par un composé de la famille des alcools.

De préférence, ledit au moins un agent de désinfection comporte de l'éthanol, par exemple de l'éthanol dilué à 70%.

De préférence, ledit au moins un agent de désinfection est appliqué à l'état liquide, notamment par pulvérisation.

Avantageusement, ledit au moins un agent de désinfection est ensuite éliminé naturellement par évaporation.

En variante, des moyens tels que de l'air chaud sont utilisés pour forcer l'évaporation et réduire le temps nécessaire à l'élimination de l'agent de désinfection.

En variante, ledit au moins un agent de désinfection pourrait être appliqué à l'état gazeux.

Pour réaliser ladite opération de désinfection selon l'invention, l'installation 10 comporte au moins un dispositif 100 de désinfection qui est agencé à l'intérieur de la première enceinte 14 de protection délimitant la première zone Z1 de l'installation 10.

Avantageusement, ledit au moins un dispositif 100 de désinfection est commandé depuis l'extérieur de l'installation 10.

Le dispositif 100 de désinfection est destiné à désinfecter au moins une partie de la roue 30 de transfert, notamment les surfaces qui sont en contact avec les récipients lors du transfert opéré, en mode de production, de l'unité 16 de soufflage vers l'unité 20 de remplissage.

Le dispositif 100 de désinfection de la roue 30 de transfert est commandé manuellement ou automatiquement depuis l'extérieur de l'installation 10 pour désinfecter, par pulvérisation d'au moins un agent de désinfection, au moins la partie 30A supérieure de transfert des récipients 12 de la roue 30.

La pulvérisation dudit au moins agent de désinfection est effectuée en mode de décontamination de l'installation 10, lorsque ladite partie 30A supérieure de transfert des récipients 12 de la roue 30 occupe la position escamotée.

Dans ce premier mode de réalisation, le dispositif 100 de désinfection comporte des moyens de pulvérisation qui sont constitués par au moins un pistolet 105 apte à pulvériser ledit au moins un agent de désinfection sur au moins ladite partie 30A supérieure de transfert des récipients 12 de la roue 30.

De préférence, le pistolet 105 est alimenté en agent de désinfection par un réservoir. L'agent de désinfection à l'état liquide est par exemple pulvérisé au moyen d'un gaz, tel que de l'air comprimé.

De préférence, le pistolet 105 est agencé à proximité des surfaces de la roue 30 de transfert à désinfecter et à l'intérieur de la première enceinte 14, dans la partie de transfert de la première zone Z1.

Avantageusement, le pistolet 105 est apte à être manipulé par un opérateur par l'intermédiaire de moyens de manipulation à distance lui permettant de réaliser ladite opération de désinfection depuis l'extérieur de l'installation 10.

Comme l'escamotage de la partie 30A supérieure de transfert des récipients de la roue 30 de transfert, l'opération de désinfection est réalisée sans qu'un opérateur ne soit à l'intérieur de l'installation 10, ne franchisse les portes 15 pour pénétrer notamment dans la partie de la première zone Z1 où se trouvent la roue 30 de transfert et ledit pistolet 105 formant le dispositif 100 de désinfection.

Tel que représenté sur la figure 2, lesdits moyens de manipulation à distance sont formés par au moins un gant 55 prolongé par une manchette et relié de manière étanche à une ouverture 57 réalisée dans la première enceinte 14 de protection.

Avantageusement, les risques de contamination liés à la présence d'un opérateur à l'intérieur de la première zone Z1 sont totalement supprimés, l'intervention de l'opérateur s'effectuant par l'intermédiaire du gant 55 à travers la première enceinte 14 mais sans jamais que ce dernier ne soit physiquement en contact avec l'air présent dans la première zone Z1.

L'opérateur ne peut dès lors pas constituer un vecteur d'introduction d'agents pathogènes susceptibles d'affecter ultérieurement les récipients 12 lors du fonctionnement en mode de production.

L'opérateur par l'intermédiaire du gant 55 est susceptible de manipuler le pistolet 105 et de pulvériser l'agent de désinfection sur les différentes surfaces à désinfecter.

Le gant 55 de manipulation permet à l'opérateur de réaliser manuellement tant l'escamotage de la partie supérieure 30A de la roue 30 de transfert (lorsque celui-ci n'est pas automatisé) que l'opération de désinfection au moyen dudit pistolet 105.

En variante, l'opération de désinfection est réalisée automatiquement et non manuellement, par exemple au moyen d'au moins un pistolet 105 déplacé automatiquement dans l'espace suivant un ou plusieurs axes par des moyens de manipulation pilotés, tel qu'un bras robotisé commandé depuis l'extérieur de l'installation 10.

L'opération de désinfection est en particulier effectuée sur au moins les moyens 36 de maintien de la partie 30A supérieure de la roue 30 de transfert et les moyens 38 de guidage associés.

Avantageusement, un dispositif 100 de désinfection tel que le pistolet 105 est facilement susceptible d'être introduit dans une installation 10 existante comme celle décrite et représentée dans le document WO-2010/081759, et cela sans nécessiter d'importantes modifications.

Bien que la mise en oeuvre de l'opération de désinfection selon le premier mode de réalisation qui vient d'être décrit puisse donner entière satisfaction, des améliorations peuvent cependant encore y être apportées.

Tout d'abord, la roue 30 de transfert demeure immobile lors de l'opération de désinfection, le seul mouvement relatif entre le pistolet 105 formant le dispositif 100 de désinfection et la roue 30 de transfert correspond dans le cas d'une réalisation manuelle à la mobilité offerte par le membre antérieur de l'opérateur en combinaison avec le gant 55.

En effet, lorsque la roue 30 de transfert est réalisée en deux parties, respectivement une partie 30A supérieure de transfert et une partie 30B inférieure d'entraînement, la première partie 30A supérieure seule est déplacée de la position de transfert vers la position escamotée et dans le mode de décontamination l'entraînement est interrompu notamment pour permettre l'escamotage.

On comprendra que l'accessibilité comme la visibilité soient moindre pour l'opérateur sur les surfaces qui sont éloignées telles que celles des moyens 38 de guidage, que sur les surfaces qui, à proximité immédiate, lui font directement face.

En conséquence, la qualité des résultats de la désinfection est susceptible d'être affectée par une application plus ou moins homogène et totale de l'agent de désinfection lorsque la désinfection est réalisée manuellement par un opérateur selon le premier mode de réalisation qui vient d'être décrit.

Le fait que l'opération de désinfection soit réalisée manuellement par un opérateur manipulant le pistolet 105 pose également la question de la fiabilité liée au facteur humain.

On comprendra qu'il ne soit pas possible de garantir totalement une parfaite répétabilité des gestes pour un même opérateur, comme entre deux opérateurs successifs, indépendamment de toute l'application dont chacun pourrait néanmoins faire preuve.

Il existe dès lors un problème de fiabilité conduisant à rechercher des solutions pour s'affranchir du facteur humain lié à toute intervention d'un opérateur.

En supprimant l'intervention manuelle d'un opérateur au cours de l'opération, le but principal poursuivit n'est pas tant de réduire les coûts d'exploitation d'une telle installation 10 mais bien d'avoir l'assurance d'une parfaite répétabilité et donc une fiabilité dans les résultats de l'opération de désinfection.

On décrira ci-après un deuxième mode de réalisation d'un dispositif 100 de désinfection pour une installation 10 de production de récipients.

Selon une première caractéristique du deuxième mode de réalisation, au moins la partie 30A supérieure de la roue 30 de transfert est susceptible d'être entrainée en rotation lorsque, en mode de décontamination, au moins ladite partie 30A occupe la position escamotée.

Par comparaison, on rappelle que dans l'installation selon le document WO-2010/081759, la partie supérieure de transfert des récipients de la roue 30 n'était pas entraînée en rotation en position escamotée.

Selon une deuxième caractéristique de ce deuxième mode de réalisation, l'opération de désinfection est réalisée entièrement de manière automatisée.

Par comparaison avec le premier mode de réalisation, toute intervention manuelle d'un opérateur est par conséquent supprimée grâce à quoi les risques précités quant à la fiabilité, la répétabilité du résultat de l'opération de désinfection, etc. liés au facteur humain sont également supprimés.

Bien entendu, les première et deuxième caractéristiques sont avantageusement combinées mais sont indépendantes l'une de l'autre.

L'entraînement en rotation de la partie supérieure de la roue 30 de transfert en position escamotée est également susceptible d'être mise en oeuvre avec un autre dispositif de désinfection, en particulier avec un pistolet 105 selon le premier mode de réalisation.

On décrira ci-après une nouvelle conception d'une roue 30 de transfert comportant notamment une partie 30A supérieure de transfert des récipients 12 qui est apte à être entraînée en rotation alors que ladite partie 30A occupe la position escamotée.

Avantageusement, au moins ladite partie 30A supérieure de transfert des récipients 12 de la roue 30 occupant ladite position escamotée est apte à effectuer un mouvement relatif par rapport au dispositif 100 de désinfection pour parfaire l'application dudit au moins un agent de désinfection lors de l'opération de désinfection.

De préférence, au moins une partie du dispositif 100 de désinfection et ladite partie 30A supérieure de transfert des récipients 12 de la roue 30 sont respectivement susceptibles d'effectuer un mouvement relatif par rapport à l'autre.

Dans ce deuxième mode réalisation, le système 50 d'entraînement de la roue 30 de transfert est apte à entraîner en rotation au moins ladite partie 30A supérieure de transfert des récipients 12 comportant les moyens 36 de maintien lorsque, en mode de décontamination, ladite partie 30A supérieure de transfert des récipients 12 occupe ladite position escamotée.

Par comparaison avec le premier mode de réalisation dans lequel le dispositif 100 de désinfection formé par le pistolet 105 était apte à être animé d'un mouvement relatif par rapport à la roue 30 de transfert, c'est dans ce deuxième mode de réalisation au moins ladite partie 30A supérieure de transfert des récipients 12 de la roue 30 qui est animée d'un mouvement relatif par rapport au dispositif 100 de désinfection.

Les figures 3 et 4 représentent de manière schématique une roue 30 de transfert, respectivement dans la position de transfert qui est occupée lorsque l'installation 10 fonctionne en mode de production de récipients 12 et dans la position escamotée qui est occupée lorsque l'installation 10 fonctionne en mode de décontamination.

Tel qu'illustré sur la figure 3, la roue 30 de transfert comporte au moins des moyens 36 de maintien pour transférer les récipients 12 à travers l'ouverture 24 ménagée dans la partie 22 commune des enceintes 14 et 18.

Les moyens 36 de maintien des récipients 12 comportent trois plateaux superposés, comme décrit précédemment dans le premier mode de réalisation. De préférence, au moins le plateau supérieur coopérant avec le col du récipient est réalisé en inox, tel que de l'inox 316L.

De préférence, la roue 30 de transfert comporte des moyens 38 de guidage intervenant de manière complémentaire auxdits moyens 36 de maintien lors du transfert des récipients 12 mais qui ne sont pas représentés sur les figures 3 et 4.

Le système 50 d'entraînement de la roue 30 de transfert des récipients 12 comporte au moins un arbre 52 primaire réalisé en au moins deux parties, respectivement une première partie 52A supérieure et une deuxième partie 52B inférieure d'entraînement, lesdites première et deuxième parties 52A, et 52B étant liées en rotation à un arbre 54 secondaire.

La première partie 52A supérieure de l'arbre 52 primaire supporte les moyens 36 de maintien avec lesquels ladite première partie 52A supérieure constitue ladite partie 30A supérieure de transfert des récipients 12 de la roue 30.

La deuxième partie 52B inférieure d'entraînement est reliée aux moyens du système 50 d'entraînement intervenant en mode de production pour entraîner, par l'intermédiaire de l'arbre 54 secondaire, ladite partie 30A supérieure de transfert des récipients 12 de la roue 30.

Les première et deuxième parties 52A et 52B de l'arbre 52 primaire sont respectivement liées en rotation à l'arbre 54 secondaire par coopération de formes.

De préférence, la liaison en rotation de l'arbre 52 primaire et de l'arbre 54 secondaire est réalisée par l'intermédiaire de moyens d'engrènement.

Les moyens d'engrènement comportent au moins des premiers moyens 56 d'engrènement intervenant pour lier en rotation l'arbre 54 secondaire avec ladite première partie 52A supérieure et des deuxièmes moyens 58 d'engrènement intervenant pour lier en rotation l'arbre 54 secondaire avec ladite deuxième partie 52B inférieure de l'arbre 52 primaire.

Avantageusement, les premiers moyens 56 d'engrènement autorisent un mouvement de giration de la première partie 52A supérieure de l'arbre 52 primaire par rapport à l'arbre 54 secondaire afin de déplacer au moins ladite partie 30A supérieure de transfert des récipients 12 entre lesdites positions de transfert et escamotée.

Comme l'illustre par comparaison les figures 3 et 4, la partie 30A supérieure de transfert des récipients effectue entre lesdites positions de transfert et escamotée un déplacement circulaire autour de l'arbre 54 secondaire.

Ce mouvement de giration correspondant à l'escamotage est rendu possible par la division en deux parties de l'arbre 52 primaire et par les premiers moyens 56 d'engrènement.

Le système 50 d'entraînement de la roue 30 de transfert comporte au moins un mécanisme 60 primaire d'embrayage.

De préférence, le mécanisme 60 primaire d'embrayage est un mécanisme de type pneumatique.

Avantageusement, le mécanisme 60 primaire d'embrayage associé à ladite deuxième partie 52B inférieure d'entraînement de l'arbre 52 primaire comporte un limiteur de couple.

De préférence, les premiers moyens 56 d'engrènement comportent au moins une roue 62 dentée qui est solidaire en rotation de la première partie 52A supérieure de l'arbre 52 primaire et une roue 64 dentée qui est solidaire en rotation du tronçon supérieur de l'arbre 54 secondaire.

La roue 62 et la roue 64 formant les premiers moyens 56 d'engrènement comportent chacune une denture externe et lesdites dentures coopèrent ensemble pour assurer une transmission du couple entre l'arbre 52 primaire et l'arbre 54 secondaire.

Avantageusement, les premiers moyens 56 d'engrènement permettent de réaliser le mouvement de giration correspondant à l'escamotage tout en maintenant la possibilité pour la partie 30A supérieure de la roue 30 de transfert d'être entraînée en rotation et notamment en position escamotée.

De préférence, les deuxièmes moyens 58 d'engrènement comportent au moins une roue 66 dentée qui est solidaire en rotation de la deuxième partie 52B inférieure de l'arbre 52 primaire et une roue 68 dentée qui est solidaire en rotation du tronçon inférieur de l'arbre 54 secondaire.

Comme les premiers moyens 56 d'engrènement, la roue 66 et la roue 68 formant les deuxièmes moyens 58 d'engrènement comportent chacune une denture externe qui coopère ensemble pour assurer une transmission du couple entre l'arbre 52 primaire et l'arbre 54 secondaire.

Le mécanisme 60 primaire d'embrayage est associé à ladite deuxième partie 52B inférieure d'entraînement de l'arbre 52 primaire pour en commander sélectivement l'entraînement, notamment par des moyens 72 motorisés.

Tel qu'indiqué précédemment pour le premier mode de réalisation, les moyens 72 motorisés sont avantageusement constitués par les moyens d'entraînement en rotation de l'unité 20 de remplissage et/ou de la deuxième roue 34 de transfert du dispositif 28 de transfert de l'installation 10.

De préférence, les moyens 72 motorisés sont liés à l'arbre 52 primaire par des moyens 74 de transmission de mouvement, tels qu'une courroie coopérant avec deux poulies 76 et 78 d'entraînement respectivement liées en rotation pour l'une 76 aux moyens 72 motorisés formant un élément menant et pour l'autre 78 à l'arbre 52 primaire formant un élément mené.

Le mécanisme 60 primaire d'embrayage est susceptible d'occuper au moins un état embrayé et un état débrayé pour transmettre sélectivement le couple lorsque la partie 30A supérieure de transfert de la roue 30 est en position de transfert, l'installation 10 fonctionnant en mode de production.

Le mécanisme 60 primaire d'embrayage occupe un état embrayé pour accoupler en rotation ladite partie 52B inférieure dudit arbre 52 primaire auxdits moyens 72 motorisés lorsque ladite au moins une partie 30A supérieure de transfert des récipients 12 de la roue 30 est en position de transfert.

Le mécanisme 60 primaire d'embrayage occupe un état débrayé pour désaccoupler ladite partie 52B inférieure dudit arbre primaire desdits moyens 72 motorisés lorsque ladite au moins une partie 30A supérieure de transfert des récipients 12 de la roue 30 est en position escamotée.

Le mécanisme 60 primaire d'embrayage est respectivement à l'état embrayé sur la figure 3 et à l'état débrayé sur la figure 4.

La roue 30 de transfert comporte un mécanisme 70 secondaire d'embrayage qui est associé à l'arbre 54 secondaire pour en commander sélectivement l'entraînement par des moyens 80 motorisés secondaire, tel qu'un moteur.

Les moyens 80 motorisés secondaire sont par exemple liés en rotation à l'arbre 54 secondaire par l'intermédiaire de moyens 82 de transmission secondaire.

Le mécanisme 70 secondaire d'embrayage est susceptible d'occuper au moins un état embrayé et un état débrayé pour transmettre sélectivement le couple lorsque la partie 30A supérieure de transfert de la roue 30 est en position escamotée, l'installation 10 fonctionnant en mode de décontamination.

Le mécanisme 70 secondaire d'embrayage occupe ledit état embrayé, illustré à la figure 4, lorsque ladite au moins une partie 30A supérieure de transfert des récipients 12 de la roue 30 est en position escamotée et cela pour accoupler en rotation, par l'intermédiaire de l'arbre 54 secondaire, ladite partie 52A supérieure dudit arbre 52 primaire auxdits moyens 80 motorisés secondaire afin d'entraîner en rotation ladite partie 30A supérieure de transfert des récipients 12.

Le mécanisme 70 secondaire d'embrayage occupe ledit état débrayé, illustré à la figure 3, lorsque ladite au moins une partie 30A supérieure de transfert des récipients 12 de la roue 30 est en position de transfert et cela pour désaccoupler ledit arbre 54 secondaire desdits moyens 80 motorisés secondaire.

Tel que représenté sur les figures 3 et 4, le mécanisme 60 primaire d'embrayage est monté sur la deuxième partie 52B inférieure d'entraînement de l'arbre 52 primaire, entre les deuxièmes moyens 58 d'engrènement avec l'arbre 54 secondaire et les moyens 72 motorisés.

Le mécanisme 70 secondaire d'embrayage est lui monté sur l'arbre 54 secondaire entre les deuxièmes 58 moyens d'engrènement avec l'arbre 52 primaire et lesdits moyens 80 motorisés secondaire.

Avantageusement, le système 50 d'entraînement constitué notamment des deux arbres 52 et 54 et des moyens d'engrènement 56 et 58 associés permet, en commandant les mécanismes 60 et 70 d'embrayage, d'assurer sélectivement l'entraînement en rotation de la partie 30A supérieure de transfert dans chacune desdites positions de transfert et escamotée.

On décrira ci-après plus particulièrement les principales étapes effectuées au niveau de la roue 30 de transfert pour réaliser une opération de désinfection entièrement automatisée selon le deuxième mode de réalisation de l'invention.

Les figures 5 à 7 représentent une roue 30 de transfert selon le deuxième mode de réalisation combinant avantageusement, d'une part, la première caractéristique concernant l'entraînement de la partie 30A supérieure en position escamotée telle que décrite et illustrée schématiquement sur les figures 3 et 4 et, d'autre part, la deuxième caractéristique concernant une automatisation de son escamotage.

Pour réaliser sélectivement l'entraînement de la partie 30A supérieure de transfert des récipients 12 dans chacune desdites positions de transfert et escamotée, le mécanisme 60 primaire et le mécanisme 70 secondaire d'embrayage de la roue 30 sont commandés de la manière décrite ci-après.

En position de transfert de la partie 30A supérieure de la roue 30 représentée à la figure 5, le couple délivré par les moyens 72 motorisés entre par la partie 52B inférieure de l'arbre 52 primaire puis, le mécanisme 60 primaire d'embrayage occupant son état embrayé, est transmis par l'intermédiaire des deuxièmes moyens 58 d'engrènement à l'arbre 54 secondaire.

L'arbre 54 secondaire transmet ensuite ce couple, par l'intermédiaire des premiers moyens 56 d'engrènement, à la partie 52A supérieure de l'arbre 52 primaire formant avec les moyens 36 de maintien ladite partie 30A supérieure de la roue 30 de transfert.

En mode de production, le mécanisme 70 secondaire d'embrayage est ouvert, occupant son état débrayé, de sorte que les moyens 80 motorisés ne sont alors pas susceptibles d'entraîner en rotation l'arbre 54 secondaire.

Pour passer du mode de fonctionnement de production au mode de décontamination, le mécanisme 60 primaire d'embrayage de type pneumatique est commandé en ouverture pour provoquer un changement d'état, de l'état embrayé vers l'état débrayé, afin d'interrompre l'entraînement en rotation par les moyens 72 motorisés de la partie 30A supérieure de la roue 30 occupant sa position de transfert.

On remarquera que les moyens 36 de maintien n'ont pas été représentés sur la partie 52A supérieure sur les figures 5 à 7.

Le mécanisme 60 primaire et le mécanisme 70 secondaire d'embrayage occupent alors respectivement leur état débrayé et la partie 30A supérieure de la roue 30 est alors libre d'être escamotée depuis l'extérieur de l'installation 10.

En effet, l'arbre 52 primaire et l'arbre 54 secondaire sont libres en rotation, les premiers moyens 56 d'engrènement et les deuxièmes moyens 58 d'engrènement ne s'opposant pas au mouvement de giration de la partie 30A supérieure de la roue 30 autour d'un axe correspondant à l'arbre 54 secondaire.

Par comparaison avec le premier mode de réalisation, l'escamotage de la partie 30A supérieure de transfert des récipients 12 de la roue 30 est avantageusement automatisé et non plus réalisé manuellement par un opérateur.

Avantageusement, la roue 30 de transfert comporte des moyens 84 d'actionnement pour déplacer automatiquement, toujours depuis l'extérieur de l'installation 10, ladite partie 30A supérieure de transfert des récipients 12 entre lesdites positions de transfert et escamotée.

De préférence et tel qu'illustré sur les figures 5 à 7, les moyens 84 d'actionnement sont constitués par au moins un vérin comportant une tige 85. La tige 85 du vérin 84 est reliée à son extrémité libre à une platine 86 qui manoeuvre la partie 52A supérieure de l'arbre 52 primaire appartenant à la partie 30A supérieure de transfert des récipients 12.

La platine 86 est traversée verticalement par la partie 52A supérieure de l'arbre 52 primaire et par l'arbre 54 secondaire, la platine 86 est susceptible de pivoter par rapport à l'arbre 54 secondaire et entraîne alors ladite partie 52A supérieure.

La partie 30A supérieure de la roue 30 de transfert comporte un support 87 fixe par rapport à la platine 86, la partie 52A supérieure et la roue 62 dentée qui se déplacent lors de la rotation de ladite partie 30A supérieure entre la position de transfert et la position escamotée.

De préférence, les moyens 38 de guidage associés aux moyens 36 de maintien de la partie 30A supérieure sont montés mobiles et escamotés automatiquement pour permettre notamment l'escamotage de la partie 30A supérieure de la roue 30 de transfert et la fermeture de l'ouverture 24 par le volet 26 d'obturation en mode de décontamination.

Les moyens 38 de guidage sont montés mobiles entre une position de guidage et une position escamotée, notamment représentées sur les figures 8 et 9 respectivement.

Avantageusement, lesdits moyens 38 de guidage sont déplacés automatiquement entre lesdites positions de guidage et escamotée par l'intermédiaire d'au moins un actionneur, tel qu'un vérin.

Dans ce deuxième mode de réalisation, les moyens 38 de guidage comportent des premiers moyens 38A de guidage et des deuxièmes moyens 38B de guidage qui interviennent respectivement lors du transfert des récipients 12 en mode de production.

De préférence, les premiers moyens 38A de guidage sont escamotés automatiquement par un vérin 88A associé et les deuxièmes moyens 38B de guidage sont escamotés par un vérin 88B associé.

Avantageusement, l'ensemble desdits actionneurs 84, 88A et 88B sont commandés depuis l'extérieur, au bénéfice de la suppression de toute présence d'un opérateur à l'intérieur de la première zone Z1 délimitée par la première enceinte 14 et permettent également de réduire les temps nécessaires aux manoeuvres d'escamotage.

Avantageusement, la roue 30 de transfert comporte des moyens 90 de verrouillage automatique qui sont commandés depuis l'extérieur pour immobiliser ladite partie 30A supérieure de transfert des récipients 12 dans la position de transfert et la position escamotée, respectivement.

Les moyens 90 de verrouillage de la roue 30 comportent au moins un organe 92 de verrouillage et un actionneur 94 associé pour déplacer sélectivement ledit organe 92 de verrouillage entre :
- une position rétractée dans laquelle au moins la partie 30A supérieure de transfert des récipients 12 de la roue 30 est libre d'être déplacée entre lesdites positions de transfert et escamotée, et
- une position engagée dans laquelle ledit organe 92 de verrouillage immobilise en position ladite partie 30A supérieure de transfert des récipients 12 de la roue 30 ou en position escamotée.

Les moyens 90 de verrouillage sont portés par la platine 86 mobile actionnée par le vérin 84 et sont donc déplacés simultanément à la partie 30A supérieure.

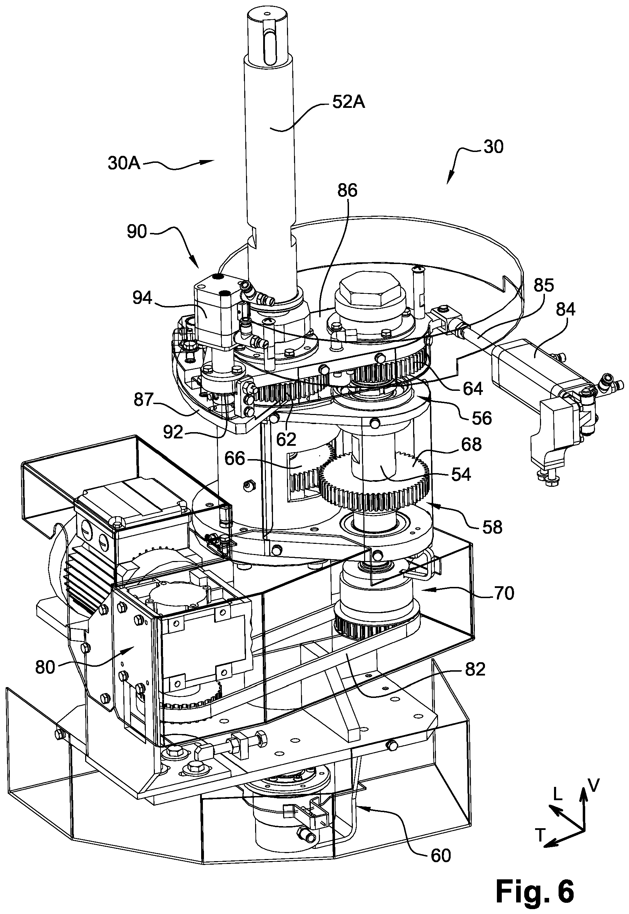

Tel qu'illustré sur les figures 5 et 6, l'organe 92 de verrouillage est introduit dans un premier logement 96 pour verrouiller ladite partie 30A supérieure en position de transfert ou dans un deuxième logement 98 pour verrouiller ladite partie 30A supérieure en position escamotée.

Les logements 96 et 98 sont portés par le support 87 qui est fixe par rapport à la platine 86 qui, déplacée par le vérin 84, porte les moyens 90 de verrouillage.

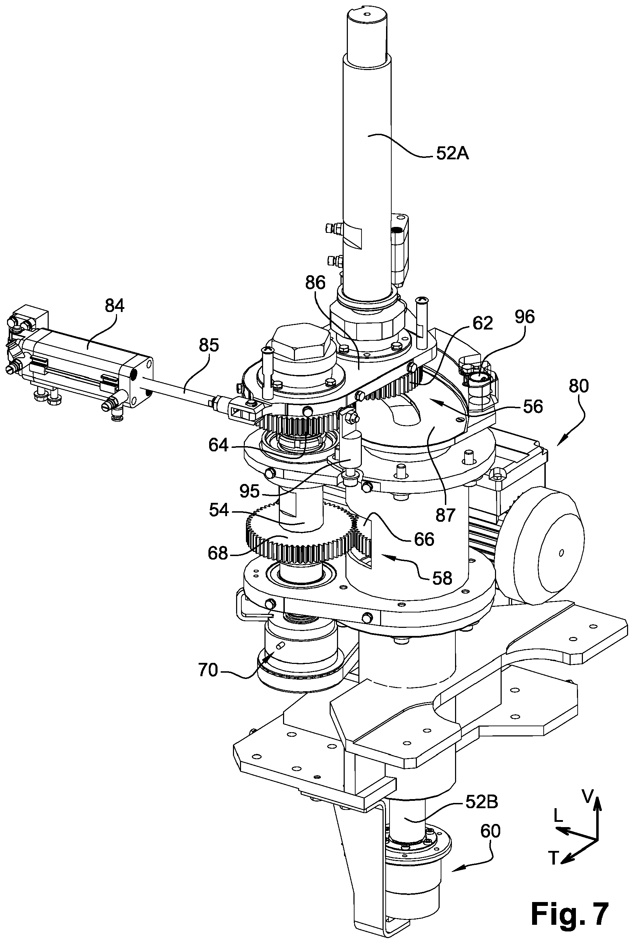

De préférence et tel que représenté sur la figure 7, des moyens 95 de butée sont prévus pour garantir un bon positionnement de la partie 30A supérieure de la roue 30 de transfert dans la position de transfert et/ou la position escamotée.

Avantageusement, les moyens 95 de butée permettent de garantir le bon positionnement vertical de l'organe 92 de verrouillage par rapport aux logements 96 et 98.

Au plus tard après que les différentes manoeuvres automatiques d'escamotage qui viennent d'être décrites aient été réalisées, on commande la fermeture du mécanisme 70 secondaire d'embrayage qui passe de l'état débrayé à l'état embrayé.

Dans l'état embrayé, le mécanisme 70 secondaire d'embrayage assure la transmission du couple des moyens 80 motorisés reçu par l'arbre 54 secondaire vers la partie 30A supérieure de la roue 30 occupant sa position escamotée grâce à quoi ladite partie 30A supérieure et plus particulièrement les moyens 36 de maintien sont entraînés en rotation lors de leur désinfection par le dispositif 100 de désinfection.

Avantageusement, les mécanismes 60 et 70 d'embrayage permettent de réaliser un ré-enclenchement automatique pour, une fois l'opération de désinfection effectuée, revenir en mode de production de l'installation 10 et cela sans nécessiter notamment l'intervention d'un opérateur.

Le mécanisme 70 secondaire d'embrayage est maintenu à l'état embrayé, soit l'état que ledit mécanisme 70 occupe au cours de l'opération de désinfection pour assurer l'entraînement en rotation des moyens 36 de maintien alors que la partie 30A supérieure est en position escamotée.

On commande en revanche la fermeture du mécanisme 60 primaire d'embrayage qui passe de l'état débrayé à l'état embrayé, état embrayé dans lequel ledit mécanisme 60 primaire d'embrayage n'est cependant pas encore accouplé.

Tel qu'indiqué précédemment, le mécanisme 60 primaire d'embrayage comporte avantageusement un limiteur de couple.

A ce stade, l'entraînement de l'arbre 54 secondaire par les moyens 80 motorisés provoque, le mécanisme 70 secondaire d'embrayage étant toujours à l'état embrayé, la transmission du couple de l'arbre 54 secondaire vers la partie 52B inférieure de l'arbre 52 primaire et cela par l'intermédiaire des deuxièmes moyens 58 d'engrènement.

Le mécanisme 60 primaire d'embrayage est alors entraîné et le couple détecté par des moyens de détection, tels qu'un capteur, associés au limiteur de couple, un signal est alors émis par les moyens de détection et exploité pour arrêter les moyens 80 motorisés et ouvrir le mécanisme 70 secondaire d'embrayage.

On a représenté aux figures 8 et 9, la roue 30 de transfert selon le deuxième mode de réalisation qui vient d'être décrit en détails en référence aux figures 5 à 7, ladite roue 30 de transfert étant illustrée respectivement en position de transfert et en position escamotée.

Tel qu'expliqué en référence aux figures 1 et 2 notamment et illustré ici sur la figure 8, la partie 30A supérieure de transfert des récipients 12 de la roue 30 s'étend, en position de transfert, à travers l'ouverture 24 ménagée dans la partie 22 commune des première et deuxième enceintes 14 et 18.

Comme on peut le voir sur la figure 8, les moyens 26 d'obturation de l'ouverture 24 sont en effet en position ouverte.

Par comparaison avec la figure 8 illustrant l'installation 10 en mode de transfert, la figure 9 illustre l'installation 10 en mode de décontamination.

Tel qu'illustré sur la figure 9, la partie 30A supérieure de la roue 30 de transfert a été escamotée automatiquement par les moyens 84 d'actionnement, les moyens 36 de maintien ne s'étendent alors plus à travers l'ouverture 24.

Les moyens de guidage 38A et 38B sont avantageusement également escamotés automatiquement par les actionneurs 88A et 88B associés.

L'ouverture 24 est alors libre d'être fermée de manière étanche par les moyens 26 d'obturation qui sont déplacés vers leur position fermée.

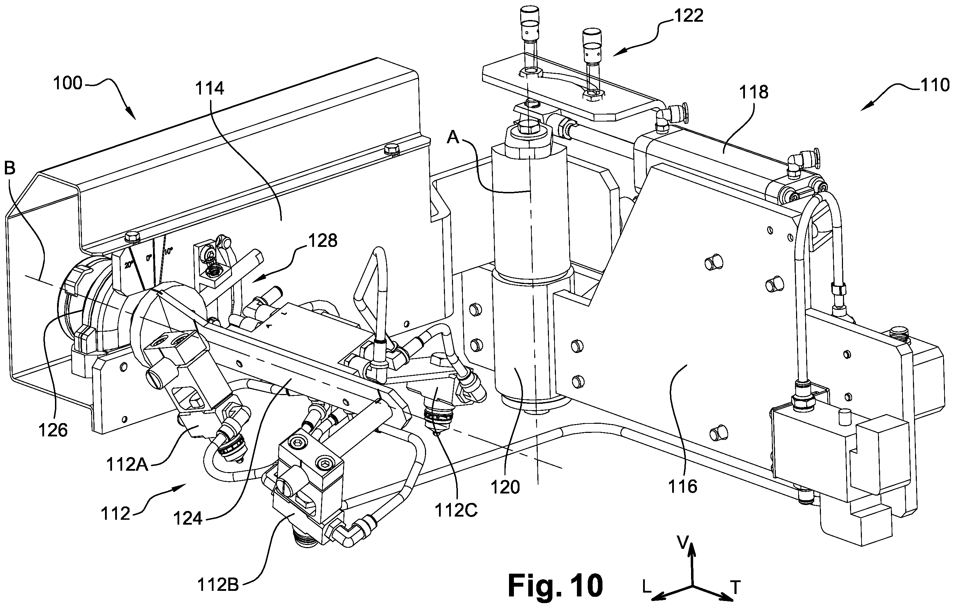

L'installation 10 est alors prête pour la mise en oeuvre de l'opération de décontamination de l'unité 20 de remplissage et également pour l'opération de désinfection au moyen du dispositif 100 de désinfection représenté sur les figures 8 et 9 et plus particulièrement visible en détails sur la figure 10.

On décrira ci-après le dispositif 100 de désinfection selon le deuxième mode de réalisation.

Le dispositif 100 de désinfection est constitué par au moins un module 110 de désinfection automatique qui, commandé depuis l'extérieur de l'installation, comporte des moyens 112 de pulvérisation d'au moins un agent de désinfection.

De préférence, ledit au moins un agent de désinfection est formé en tout ou en partie par un composé de la famille des alcools, tel que de l'éthanol dilué à 70%, qui est pulvérisé à l'état liquide par les moyens 112 de pulvérisation du module 110 pour désinfecter au moins ladite partie 30A supérieure de transfert des récipients 12 de la roue 30.

De préférence, ledit au moins un agent de désinfection est ensuite éliminé naturellement par évaporation.

Avantageusement, le module 110 de désinfection automatique est apte à désinfecter au moins ladite partie 30A supérieure de transfert des récipients 12 de la roue 30 de transfert.

De préférence, le module 110 de désinfection automatique est destiné à désinfecter au moins les moyens 36 de maintien, tout particulièrement le plateau supérieur en contact avec le col du récipient, et les moyens 38 de guidage.

Avantageusement, le module 110 de désinfection automatique est monté mobile entre au moins une position de désinfection et une position de repos.

Selon sa conception et son agencement dans l'installation 10, le module 110 de désinfection automatique est monté mobile en translation et/ou en rotation pour être déplacé entre lesdites positions de désinfection et de repos.

Le module 110 de désinfection automatique occupe, en mode de décontamination, la position de désinfection qui correspond à une position dans laquelle lesdits moyens 112 de pulvérisation sont aptes à désinfecter au moins la partie 30A supérieure de transfert des récipients 12 de la roue 30.

Le module 110 de désinfection automatique occupe, en mode de production, la position de repos qui correspond à une position dans laquelle le dispositif 100 de désinfection est escamoté.

Avantageusement, l'escamotage du dispositif 110 de désinfection automatique permet notamment d'éviter de perturber l'écoulement de l'air en cours de production lorsque l'installation 10 comporte un système d'insufflation d'air filtré pour établir une surpression à l'intérieur des enceintes 14 et 18 afin de limiter les risques de contamination.

En position escamotée, le dispositif 110 de désinfection automatique est maintenu hors du parcours suivi par les récipients 12 en mode de production de l'installation 10 ce qui permet de prévenir certains risques vis-à-vis des récipients 12 par exemple en cas de fuite.

De préférence et tel qu'illustré sur les figures 8 et 9, le module 110 de désinfection automatique est relié à ladite partie 22 commune desdites première et deuxième enceintes 14 et 18.

Avantageusement, le module 110 de désinfection automatique est agencé verticalement en hauteur par rapport à la roue 30 de transfert, c'est-à-dire verticalement au-dessus sans pour autant être nécessairement à l'aplomb de la roue 30.

Grâce à un tel agencement du module 110 de désinfection par rapport à la roue 30 de transfert, ledit au moins agent de désinfection est en position de désinfection pulvérisé par lesdits moyens 112 de pulvérisation en direction d'au moins de la partie 30A supérieure de transfert des récipients 12 de la roue 30 située verticalement en dessous.

Le module 110 de désinfection automatique comporte au moins un bras 114 desdits moyens 112 de pulvérisation dudit au moins un agent de désinfection.

Le bras 114 est monté mobile par rapport à un châssis 116 du module pour être déployé vers la position de désinfection ou replié vers la position de repos, le châssis 116 fixe est relié à la partie 22 commune.

Le bras 114 est apte à être déplacé par au moins un actionneur 118 qui est commandé sélectivement en fonction du mode de fonctionnement, de production ou de décontamination, de l'installation 10.

De préférence, le bras 114 est monté mobile en rotation autour d'un pivot 120 qui, déterminant un axe A de rotation, est solidaire du châssis 116 fixe du module 110.

Le bras 114 pivote autour dudit pivot 120 entre au moins une première position correspondant à la position de désinfection du module 110 et une deuxième position correspond à la position de repos du module 110.

Le bras 114 pivote ici à 90° par rapport au châssis 116 entre lesdites première et deuxième positions respectivement représentées sur les figures 8 et 9.

De préférence, des moyens 122 de butée associés à l'actionneur 118 déterminent lesdites première et deuxième positions du bras 114.

Les moyens 112 de pulvérisation sont fixés sur un support 124 qui est avantageusement monté mobile par rapport au bras 114 qui est déplacé par l'actionneur 118 entre les positions de désinfection et de repos.

De préférence, le support 124 s'étend orthogonalement, à 90°, à l'extrémité libre du bras 114.

Le support 124 est monté mobile en rotation par rapport au bras 114 autour d'un axe B de rotation orthogonal au bras 114 qui est lui-même monté mobile en rotation autour de l'axe A par rapport au châssis 116.

Le support 124 comportant les moyens 112 de pulvérisation est apte à être entraîné en rotation autour dudit axe B par des moyens 126 d'entraînement, tels qu'un moteur.

Avantageusement, lesdits moyens 112 de pulvérisation de l'agent de désinfection sont déplacés par le support 124 autour de l'axe B, respectivement entre au moins une première position angulaire P1 et une deuxième position angulaire P2.

De préférence, la première position angulaire P1 correspond par exemple à un angle de 20° par rapport à un plan de référence à 0° correspondant à un plan vertical d'orientation transversale et la deuxième position angulaire P2 à un angle de 10° par rapport audit plan de référence, lesdites positions P1 et P2 étant atteinte par des rotations de sens opposé par rapport audit plan de référence.

Avantageusement, la rotation du support 124 permet de réaliser dans ladite position de désinfection un balayage lors de l'opération de désinfection d'au moins ladite partie 30A supérieure de transfert des récipients 12 de la roue 30.

De préférence, des moyens 128 de butée sont associés au moteur 126 et déterminent lesdites première position angulaire P1 et une deuxième position angulaire P2.

Les moyens 112 de pulvérisation du module 110 formant le dispositif 100 de désinfection comportent au moins une buse apte à pulvériser ledit au moins un agent de désinfection.

Tel que représenté sur les figures 10 à 12, le module 110 de désinfection automatique comporte au moins trois buses référencées respectivement 112A, 112B et 112C.

La position de chaque buse est également réglable dans au moins une direction de l'espace défini par le trièdre (L, V, T) et en particulier réglable par rapport au support 124.

Les buses 112A, 112B et 112C sont positionnées en fonction des surfaces à désinfecter, au moins l'une des buses formant par exemple des premiers moyens de pulvérisation pour désinfecter lesdits moyens 36 de maintien tandis qu'au moins une autre buse forme des deuxièmes moyens de pulvérisation pour désinfecter lesdits moyens 38A et 38B de guidage.

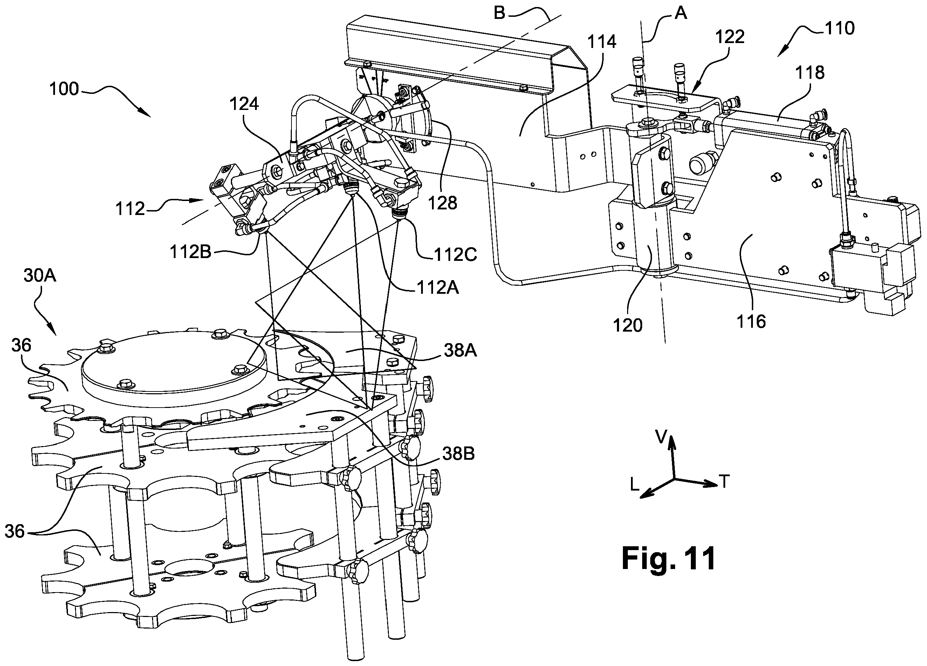

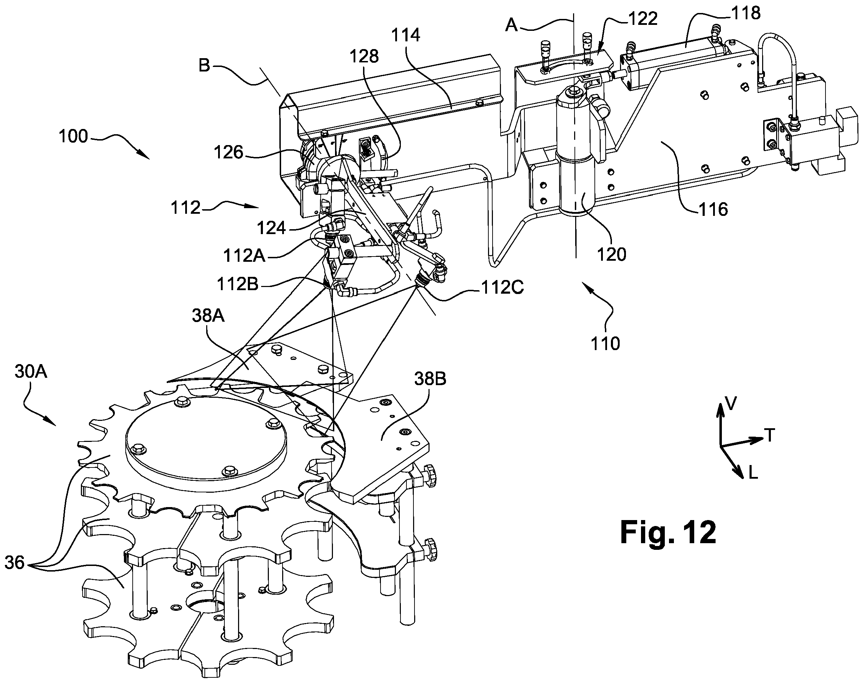

Les figures 11 et 12 illustrent plus particulièrement les jets qui sont pulvérisés par lesdites buses 112A, 112B et 112C formant les moyens 112 de pulvérisation du module 110.

Les figures 11 et 12 représentent le module 110 de désinfection automatique en position de désinfection, le bras 114 déployé est aligné transversalement avec le châssis 116 et le support 124 des moyens 112 de pulvérisation s'étend alors orthogonalement selon la direction longitudinale.

Sur la figure 11, le support 124 se trouve dans la première position angulaire P1, c'est-à-dire incliné vers l'avant d'un angle de 10° par rapport audit plan vertical de référence.

Sur la figure 12, le support 124 se trouve dans la deuxième position angulaire P2, c'est-à-dire incliné vers l'arrière d'un angle de 20° par rapport audit plan vertical de référence.

Dans la première position angulaire P1, les moyens 112 de pulvérisation désinfectent principalement les moyens 36 de maintien de la roue 30 de transfert tandis que, dans la deuxième position P2, les moyens 112 de pulvérisation désinfectent principalement les moyens 38 de guidage formés ici des premiers moyens 38A et des deuxièmes moyens 38B de guidage.

Les jets d'agent de désinfection pulvérisés par les buses 112A, 112B et 112C sont de préférence des jets globalement plats comme l'illustrent les figures 11 et 12.

Lors de l'opération de désinfection réalisée automatiquement avec le module 110 de désinfection, la partie 30A supérieure de la roue et donc les moyens 36 de maintien formés par les trois plateaux superposés se déplacent relativement par rapport au dispositif 100 de désinfection.

Tel que décrit précédemment, les moyens 36 de maintien sont avantageusement entraînés en rotation par les moyens 80 motorisés par l'intermédiaire de l'arbre 54 secondaire, du mécanisme 70 secondaire d'embrayage à l'état embrayé et des moyens 56 d'engrènement intervenant entre la partie 52A supérieure de l'arbre 52 primaire et l'arbre 54 secondaire.

Grâce à l'entraînement en rotation de la partie supérieure 30A de la roue 30, les moyens 36 de maintien se déplacent relativement au dispositif 100 de désinfection permettant d'obtenir une parfaite application dudit au moins un agent de désinfection sur l'ensemble des moyens 36 de maintien.

En variante non représentée, le module 110 de désinfection automatique est monté mobile en translation par rapport à la roue 30 de transfert, notamment entre les positions de repos et de désinfection.

Le module 110 comporte par exemple un chariot mû par des moyens d'entraînement pour coulisser, parallèlement à ladite partie 22 commune desdites première et deuxième enceintes 14 et 18 afin d'être avancé ou reculé par rapport à la roue 30 de transfert.

Avantageusement, les moyens 112 de pulvérisation peuvent également être montés mobiles, indépendamment du déplacement du module 110 de désinfection.

Selon la variante, les moyens d'entraînement du chariot du module 110 de désinfection automatique sont aptes à entraîner en déplacement les moyens 26 d'obturation de l'ouverture 24 ou réciproquement de sorte que le déplacement dudit module 110 de désinfection soit avantageusement réalisé simultanément à l'ouverture et la fermeture de l'ouverture 24 par les moyens 26 d'obturation.

## Revendications

1. Installation (10) pour la production de récipients (12), notamment de bouteilles, comportant au moins :
- une première enceinte (14) de protection délimitant une première zone (Z1) à l'intérieur de laquelle est agencée au moins une unité (16) de soufflage de récipients,
- une deuxième enceinte (18) de confinement qui, au moins en partie accolée à la première enceinte (14) attenante par une partie (22) commune, délimite une deuxième zone (Z2) stérile à l'intérieur de laquelle est agencée au moins une unité (20) de remplissage des récipients (12) fabriqués,
- au moins une ouverture (24) qui, réalisée dans ladite partie (22) commune desdites première et deuxième enceintes (14, 18), est destinée à permettre le transfert des récipients (12) de l'unité (16) de soufflage à l'unité (20) de remplissage,
- des moyens (26) d'obturation de ladite ouverture (24) qui sont aptes à être déplacés sélectivement entre une position ouverte dans laquelle les moyens (26) d'obturation autorisent ledit transfert des récipients (12) à travers l'ouverture (24) et une position fermée dans laquelle les moyens (26) d'obturation interdisent tout transfert en isolant la deuxième enceinte (18) pour réaliser une décontamination de l'unité (20) de remplissage,
- un dispositif (28) de transfert comportant au moins une roue (30) de transfert qui est adjacente à ladite ouverture (24) pour transférer les récipients (12) entre l'unité (16) de soufflage et l'unité (20) de remplissage,
ladite roue (30) de transfert comportant au moins une partie (30A) supérieure de transfert des récipients (12) qui est montée mobile entre au moins une première position de transfert occupée lorsque l'installation (10) est dans un mode de production et une deuxième position escamotée occupée lorsque l'installation (10) est dans un mode de décontamination, le déplacement de ladite partie (30A) supérieure de la roue (30) montée mobile entre lesdites positions de transfert et escamotée étant commandé depuis l'extérieur de l'installation (10), et
- un système (50) d'entraînement de la roue (30) de transfert pour entraîner en rotation au moins la partie (30A) supérieure de transfert des récipients (12) de la roue (30) lorsque, en mode de production, ladite partie (30A) supérieure occupe ladite position de transfert,
**caractérisée en ce que** l'installation (10) comporte un dispositif (100) de désinfection de la roue (30) de transfert qui est commandé depuis l'extérieur de l'installation (10) pour désinfecter, par pulvérisation d'au moins un agent de désinfection, au moins la partie (30A) supérieure de transfert des récipients (12) de la roue (30) lorsque, en mode de décontamination, ladite partie (30A) supérieure de transfert des récipients (12) de la roue (30) occupe la position escamotée.

2. Installation selon la revendication 1, **caractérisée en ce que** le dispositif (100) de désinfection est constitué par au moins un pistolet (105) agencé à l'intérieur de l'installation (10) et apte à être manipulé par un opérateur depuis l'extérieur de l'installation, par l'intermédiaire de moyens (55) de manipulation à distance, pour pulvériser ledit au moins un agent de désinfection sur au moins ladite partie (30A) supérieure de transfert des récipients (12) de la roue (30).

3. Installation selon la revendication 1, **caractérisée en ce que** le dispositif (100) de désinfection est constitué par au moins un module (110) de désinfection automatique qui, commandé depuis l'extérieur de l'installation (10), comporte des moyens (112) de pulvérisation dudit au moins un agent de désinfection pour désinfecter au moins ladite partie (30A) supérieure de transfert des récipients (12) de la roue (30).

4. Installation selon la revendication 3, **caractérisée en ce que** ledit module (110) de désinfection automatique est monté mobile entre au moins :
- une position de désinfection, occupée en mode de décontamination, dans laquelle lesdits moyens (112) de pulvérisation sont aptes à désinfecter au moins la partie (30A) supérieure de transfert des récipients (12) de la roue (30) ; et
- une position de repos, occupée en mode de production, dans laquelle ledit module (110) de désinfection est escamoté.

5. Installation selon la revendication 4, **caractérisée en ce que** le module (110) de désinfection automatique comporte au moins un bras (114) desdits moyens (112) de pulvérisation de l'agent de désinfection et **en ce que** ledit bras (114) est déplacé sélectivement par au moins un actionneur (118) pour être déployé vers la position de désinfection ou escamoté vers la position de repos.

6. Installation selon l'une quelconque des revendications 4 ou 5, **caractérisée en ce que** lesdits moyens (112) de pulvérisation de l'agent de désinfection sont montés mobiles en rotation autour d'un axe (B), respectivement entre au moins une première position angulaire et une deuxième position angulaire, pour réaliser dans ladite position de désinfection un balayage d'au moins ladite partie (30A) supérieure de transfert des récipients (12) de la roue (30).

7. Installation selon l'une quelconque des revendications 4 à 6, **caractérisée en ce que** ladite partie (30A) supérieure de transfert des récipients (12) de la roue (30) comporte au moins des moyens (36) de maintien auxquels sont associés des moyens (38) de guidage qui, en mode de production, participent respectivement au transfert des récipients (12) et **en ce que** le module (110) de désinfection automatique comporte au moins des premiers moyens de pulvérisation pour désinfecter lesdits moyens (36) de maintien et des deuxièmes moyens de pulvérisation pour désinfecter lesdits moyens (38) de guidage.

8. Installation selon l'une quelconque des revendications 1 à 7, **caractérisée en ce que** ledit au moins un agent de désinfection est formé en tout ou en partie par un composé de la famille des alcools qui est pulvérisé à l'état liquide par le dispositif (100) de désinfection pour désinfecter au moins ladite partie (30A) supérieure de transfert des récipients (12) de la roue (30).

9. Installation selon l'une quelconque des revendications 1 à 8, **caractérisée en ce que** au moins une partie du dispositif (100) de désinfection ou la partie (30A) supérieure de transfert des récipients (12) de la roue (30) occupant ladite position escamotée est apte à effectuer un mouvement relatif par rapport à l'autre pour parfaire l'application dudit au moins un agent de désinfection.

10. Installation selon l'une quelconque des revendications 1 à 9, **caractérisée en ce que** ledit système (50) d'entraînement de la roue (30) de transfert est apte à entraîner en rotation au moins ladite partie (30A) supérieure de transfert des récipients (12) de la roue (30) lorsque, en mode de décontamination, ladite partie (30A) supérieure de transfert des récipients (12) occupe ladite position escamotée,

11. Installation selon la revendication 10, **caractérisée en ce que** ledit système (50) d'entraînement de la roue (30) de transfert des récipients (12) comporte au moins un arbre (52) primaire réalisé en au moins deux parties, respectivement une première partie (52A) supérieure à laquelle est reliée au moins ladite partie (30A) supérieure de transfert des récipients (12) et une deuxième partie (52B) inférieure d'entraînement, lesdites première et deuxième parties (52A, 52B) étant liées en rotation à un arbre (54) secondaire.

12. Installation selon la revendication 11, **caractérisée en ce que** les première et deuxième parties (52A, 52B) de l'arbre (52) primaire sont respectivement liées en rotation à l'arbre (54) secondaire par coopération de formes, de préférence ladite liaison en rotation est réalisée par engrènement.

13. Installation selon l'une des revendications 11 ou 12, **caractérisée en ce que** un mécanisme (70) secondaire d'embrayage est associé à l'arbre (54) secondaire pour en commander sélectivement l'entraînement par des moyens (80) motorisés.

14. Installation selon la revendication 13, **caractérisée en ce que** ledit mécanisme (70) secondaire d'embrayage occupe au moins :
- un état embrayé lorsque ladite au moins une partie (30A) supérieure de transfert des récipients (12) de la roue (30) est en position escamotée pour accoupler en rotation, par l'intermédiaire de l'arbre (54) secondaire, ladite partie (52A) supérieure dudit arbre (52) primaire auxdits moyens (80) motorisés afin d'entraîner en rotation ladite partie (30A) supérieure de transfert des récipients (12), et
- un état débrayé lorsque ladite au moins une partie (30A) supérieure de transfert des récipients (12) de la roue (30) est en position de transfert pour désaccoupler ledit arbre (54) secondaire desdits moyens (80) motorisés.

## Patentansprüche

1. Anlage (10) zur Herstellung von Behältern (12), insbesondere von Flaschen, welche wenigstens umfasst:
- einen ersten Schutzraum (14), der einen ersten Bereich (Z1) begrenzt, in dessen Innerem wenigstens eine Einheit (16) zum Blasen von Behältern angeordnet ist,
- einen zweiten Einschließungsraum (18), welcher wenigstens teilweise an den angrenzenden ersten Raum (14) durch einen gemeinsamen Teil (22) angefügt ist und einen zweiten sterilen Bereich (Z2) begrenzt, in dessen Innerem wenigstens eine Einheit (20) zum Füllen der hergestellten Behälter (12) angeordnet ist,
- wenigstens eine Öffnung (24), welche in dem gemeinsamen Teil (22) des ersten und des zweiten Raumes (14, 18) hergestellt ist und dazu bestimmt ist, den Transfer der Behälter (12) von der Blaseinheit (16) zur Fülleinheit (20) zu ermöglichen,
- Mittel (26) zum Verschließen der Öffnung (24), welche geeignet sind, selektiv verschoben zu werden zwischen einer geöffneten Position, in welcher die Mittel (26) zum Verschließen den Transfer der Behälter (12) durch die Öffnung (24) hindurch zulassen, und einer geschlossenen Position, in welcher die Mittel (26) zum Verschließen jeden Transfer unmöglich machen, indem sie den zweiten Raum (18) isolieren, um eine Dekontamination der Fülleinheit (20) durchzuführen,
- eine Transfervorrichtung (28), die wenigstens ein Transferrad (30) aufweist, welches der Öffnung (24) benachbart ist, um einen Transfer der Behälter (12) zwischen der Blaseinheit (16) und der Fülleinheit (20) durchzuführen,
wobei das Transferrad (30) wenigstens einen oberen Teil (30A) zum Transfer der Behälter (12) aufweist, welcher beweglich zwischen wenigstens einer ersten Transferposition, die eingenommen wird, wenn sich die Anlage (10) in einer Produktions-Betriebsart befindet, und einer zweiten, eingezogenen Position, die eingenommen wird, wenn sich die Anlage (10) in einer Dekontaminations-Betriebsart befindet, angebracht ist, wobei die Verschiebung des oberen Teils (30A) des Rades (30), der zwischen der Transfer- und der eingezogenen Position beweglich angebracht ist, von außerhalb der Anlage (10) gesteuert wird, und
- ein System (50) zum Antrieb des Transferrades (30), um wenigstens den zum Transfer der Behälter (12) bestimmten oberen Teil (30A) des Rades (30) drehend anzutreiben, wenn in der Produktions-Betriebsart der obere Teil (30A) die Transferposition einnimmt,
**dadurch gekennzeichnet, dass** die Anlage (10) eine Vorrichtung (100) zur Desinfektion des Transferrades (30) aufweist, welche von außerhalb der Anlage (10) gesteuert wird, um durch Zerstäubung wenigstens eines Desinfektionsmittels wenigstens den zum Transfer der Behälter (12) bestimmten oberen Teil (30A) des Rades (30) zu desinfizieren, wenn in der Dekontaminations-Betriebsart der zum Transfer der Behälter (12) bestimmte obere Teil (30A) des Rades (30) die eingezogene Position einnimmt.

2. Anlage nach Anspruch 1, **dadurch gekennzeichnet, dass** die Vorrichtung (100) zur Desinfektion aus wenigstens einer Pistole (105) besteht, die im Inneren der Anlage (10) angeordnet ist und geeignet ist, durch einen Bediener von außerhalb der Anlage über Mittel (55) zur Fernhandhabung gehandhabt zu werden, um das wenigstens eine Desinfektionsmittel auf wenigstens dem zum Transfer der Behälter (12) bestimmten oberen Teil (30A) des Rades (30) zu zerstäuben.

3. Anlage nach Anspruch 1, **dadurch gekennzeichnet, dass** die Vorrichtung (100) zur Desinfektion aus wenigstens Modul (110) zur automatischen Desinfektion besteht, welches von außerhalb der Anlage (10) gesteuert wird und Mittel (112) zur Zerstäubung des wenigstens einen Desinfektionsmittels aufweist, um wenigstens den zum Transfer der Behälter (12) bestimmten oberen Teil (30A) des Rades (30) zu desinfizieren.

4. Anlage nach Anspruch 3, **dadurch gekennzeichnet, dass** das Modul (110) zur automatischen Desinfektion beweglich angebracht ist zwischen:
- einer in der Dekontaminations-Betriebsart eingenommenen Desinfektionsposition, in welcher die Mittel (112) zur Zerstäubung in der Lage sind, wenigstens den zum Transfer der Behälter (12) bestimmten oberen Teil (30A) des Rades (30) zu desinfizieren; und
- einer in der Produktions-Betriebsart eingenommenen Ruheposition, in welcher das Desinfektionsmodul (110) eingezogen ist.

5. Anlage nach Anspruch 4, **dadurch gekennzeichnet, dass** das Modul (110) zur automatischen Desinfektion wenigstens einen Arm (114) der Mittel (112) zur Zerstäubung des Desinfektionsmittels aufweist, und dadurch, dass der Arm (114) selektiv durch wenigstens einen Aktuator (118) bewegt wird, um in die Desinfektionsposition ausgefahren oder in die Ruheposition eingezogen zu werden.

6. Anlage nach einem der Ansprüche 4 oder 5, **dadurch gekennzeichnet, dass** die Mittel (112) zur Zerstäubung des Desinfektionsmittels drehbeweglich um eine Achse (B) jeweils zwischen wenigstens einer ersten Winkelposition und einer zweiten Winkelposition angebracht sind, um in der Desinfektionsposition eine Überstreichung wenigstens des zum Transfer der Behälter (12) bestimmten oberen Teils (30A) des Rades (30) durchzuführen.

7. Anlage nach einem der Ansprüche 4 bis 6, **dadurch gekennzeichnet, dass** der zum Transfer der Behälter (12) bestimmte obere Teil (30A) des Rades (30) wenigstens Haltemittel (36) aufweist, denen Führungsmittel (38) zugeordnet sind, welche in der Produktions-Betriebsart jeweils am Transfer der Behälter (12) beteiligt sind, und dadurch, dass das Modul (110) zur automatischen Desinfektion wenigstens erste Zerstäubungsmittel zum Desinfizieren der Haltemittel (36) und zweite Zerstäubungsmittel zum Desinfizieren der Führungsmittel (38) aufweist.

8. Anlage nach einem der Ansprüche 1 bis 7, **dadurch gekennzeichnet, dass** das wenigstens eine Desinfektionsmittel ganz oder teilweise von einer Verbindung aus der Familie der Alkohole gebildet wird, welche von der Desinfektionsvorrichtung (100) im flüssigen Zustand zerstäubt wird, um wenigstens den zum Transfer der Behälter (12) bestimmten oberen Teil (30A) des Rades (30) zu desinfizieren.

9. Anlage nach einem der Ansprüche 1 bis 8, **dadurch gekennzeichnet, dass** wenigstens ein Teil der Desinfektionsvorrichtung (100) oder der die eingezogene Position einnehmende, zum Transfer der Behälter (12) bestimmte obere Teil (30A) des Rades (30) in der Lage ist, eine relative Bewegung bezüglich des anderen auszuführen, um die Anwendung des wenigstens einen Desinfektionsmittels zu vervollkommnen.

10. Anlage nach einem der Ansprüche 1 bis 9, **dadurch gekennzeichnet, dass** das System (50) zum Antrieb des Transferrades (30) geeignet ist, wenigstens den zum Transfer der Behälter (12) bestimmten oberen Teil (30A) des Rades (30) drehend anzutreiben, wenn in der Dekontaminations-Betriebsart der zum Transfer der Behälter (12) bestimmte obere Teil (30A) die eingezogene Position einnimmt.

11. Anlage nach Anspruch 10, **dadurch gekennzeichnet, dass** das System (50) zum Antrieb des Transferrades (30) der Behälter (12) wenigstens eine primäre Welle (52) aufweist, die aus wenigstens zwei Teilen hergestellt ist, nämlich einem oberen, ersten Teil (52A), mit dem wenigstens der zum Transfer der Behälter (12) bestimmte obere Teil (30A) verbunden ist, und einem unteren, zweiten Antriebsteil (52B), wobei der erste und der zweite Teil (52A, 52B) mit einer sekundären Welle (54) drehfest verbunden sind.

12. Anlage nach Anspruch 11, **dadurch gekennzeichnet, dass** der erste und der zweite Teil (52A, 52B) der primären Welle (52) jeweils mit der sekundären Welle (54) durch Formschluss drehfest verbunden sind, wobei die drehfeste Verbindung vorzugsweise durch Eingriff hergestellt wird.

13. Anlage nach einem der Ansprüche 11 oder 12, **dadurch gekennzeichnet, dass** der sekundären Welle (54) ein sekundärer Kupplungsmechanismus (70) zugeordnet ist, um ihren Antrieb selektiv durch motorisch angetriebene Mittel (80) zu steuern.

14. Anlage nach Anspruch 13, **dadurch gekennzeichnet, dass** der sekundäre Kupplungsmechanismus (70) wenigstens einnimmt:
- einen eingekuppelten Zustand, wenn sich der wenigstens eine zum Transfer der Behälter (12) bestimmte obere Teil (30A) des Rades (30) in der eingezogenen Position befindet, um über die sekundäre Welle (54) den oberen Teil (52A) der primären Welle (52) mit den motorisch angetriebenen Mitteln (80) drehfest zu kuppeln, um den zum Transfer der Behälter (12) bestimmten oberen Teil (30A) drehend anzutreiben, und
- einen ausgekuppelten Zustand, wenn sich der wenigstens eine zum Transfer der Behälter (12) bestimmte obere Teil (30A) des Rades (30) in der Transferposition befindet, um die sekundäre Welle (54) von den motorisch angetriebenen Mitteln (80) abzukuppeln.

## Claims

1. Installation (10) for producing containers (12), in particular bottles, including at least:
- a first protective enclosure (14) delimiting a first zone (Z1) inside which is arranged at least one unit (16) for blow moulding containers,
- a second confinement enclosure (18) that, at least in the part attached to the first adjacent enclosure (14) by a common part (22), delimits a second sterile zone (Z2) inside which is arranged at least one unit (20) for filling manufactured containers (12),
- at least one opening (24) that, made in said common part (22) of said first and second enclosures (14, 18), is designed to make possible the transfer of the containers (12) from the blow-moulding unit (16) to the filling unit (20),
- means (26) for sealing said opening (24) that are able to be moved selectively between an open position in which the sealing means (26) allow said transfer of the containers (12) through the opening (24) and a closed position in which the sealing means (26) prevent any transfer by isolating the second enclosure (18) to carry out a decontamination of the filling unit (20),
- a transfer device (28) that includes at least one transfer wheel (30) that is adjacent to said opening (24) for transferring the containers (12) between the blow-moulding unit (16) and the filling unit (20),
said transfer wheel (30) including at least one upper part (30A) for transfer of the containers (12) that is mounted to move between at least one first transfer position that is occupied when the installation (10) is in a production mode and a second retracted position that is occupied when the installation (10) is in a decontamination mode, the movement of said upper part (30A) of the wheel (30) mounted to move between said transfer and retracted positions being controlled from outside the installation (10), and
- a drive system (50) of the transfer wheel (30) for driving in rotation at least the upper part (30A) for transfer of the containers (12) from the wheel (30) when, in the production mode, said upper part (30A) occupies said transfer position, **characterised in that** the installation (10) includes a device (100) for disinfecting the transfer wheel (30) that is controlled from outside the installation (10) for disinfecting, by spraying at least one disinfecting agent, at least the upper part (30A) for transfer of the containers (12) from the wheel (30) when, in the decontamination mode, said upper part (30A) for transfer of the containers (12) from the wheel (30) occupies the retracted position.

2. Installation according to claim 1, **characterised in that** the disinfecting device (100) consists of at least one gun (105) arranged inside the installation (10) and able to be operated by an operator from outside the installation, by means of remote handling means (55), for spraying said at least one disinfecting agent on at least said upper part (30A) for transfer of the containers (12) from the wheel (30).

3. Installation according to claim 1, **characterised in that** the disinfecting device (100) consists of at least one automatic disinfecting module (110) that, controlled from outside the installation (10), includes means (112) for spraying said at least one disinfecting agent to disinfect at least said upper part (30A) for transfer of the containers (12) from the wheel (30).

4. Installation according to claim 3, **characterised in that** said automatic disinfecting module (110) is mounted to move between at least:
- a disinfection position, occupied in the decontamination mode, in which said spraying means (112) are able to disinfect at least the upper part (30A) for transfer of the containers (12) from the wheel (30); and
- a rest position, occupied in the production mode, in which said disinfecting module (110) is retracted.

5. Installation according to claim 4, **characterised in that** the automatic disinfecting module (110) includes at least one arm (114) of said means (112) for spraying the disinfecting agent and **in that** said arm (114) is moved selectively by at least one actuator (118) to be deployed toward the disinfection position or retracted toward the rest position.

6. Installation according to either one of claims 4 or 5, **characterised in that** said means (112) for spraying the disinfecting agent are mounted to move in rotation around an axis (B), respectively between at least a first angular position and a second angular position, for carrying out in said disinfection position a sweeping action of at least said upper part (30A) for transfer of the containers (12) from the wheel (30).

7. Installation according to any one of claims 4 to 6, **characterised in that** said upper part (30A) for transfer of the containers (12) from the wheel (30) includes at least holding means (36) with which are associated guide means (38) that, in the production mode, participate respectively in the transfer of the containers (12), and **in that** the automatic disinfecting module (110) includes at least first spraying means for disinfecting said holding means (36) and second spraying means for disinfecting said guide means (38).

8. Installation according to any one of claims 1 to 7, **characterised in that** said at least one disinfecting agent is formed completely or in part by a compound of the alcohol family that is sprayed in the liquid state by the disinfecting device (100) for disinfecting at least said upper part (30A) for transfer of the containers (12) from the wheel (30).

9. Installation according to any one of claims 1 to 8, **characterised in that** at least one part of the disinfecting device (100) or the upper part (30A) for transfer of the containers (12) from the wheel (30) occupying said retracted position is able to carry out one relative movement in relation to the next to improve the application of said at least one disinfecting agent.

10. Installation according to any one of claims 1 to 9, **characterised in that** said drive system (50) of the transfer wheel (30) is able to drive in rotation at least said upper part (30A) for transfer of the containers (12) from the wheel (30) when, in the decontamination mode, said upper part (30A) for transfer of the containers (12) occupies said retracted position.

11. Installation according to claim 10, **characterised in that** said drive system (50) of the transfer wheel (30) of the containers (12) includes at least one primary shaft (52) made in at least two parts, respectively a first upper drive part (52A) to which is connected at least said upper part (30A) for transfer of the containers (12) and a second lower drive part (52B), said first and second parts (52A, 52B) being coupled in rotation to a secondary shaft (54).

12. Installation according to claim 11, **characterised in that** the first and second parts (52A, 52B) of the primary shaft (52) are respectively coupled in rotation to the secondary shaft (54) by mating shapes; said coupling in rotation is preferably made by meshing.

13. Installation according to either one of claims 11 or 12, **characterised in that** a secondary clutch mechanism (70) is associated with the secondary shaft (54) to control its driving selectively by motorized means (80).

14. Installation according to claim 13, **characterised in that** said secondary clutch mechanism (70) occupies at least:
- an engaged state when said at least one upper part (30A) for transfer of the containers (12) from the wheel (30) is in the retracted position for coupling in rotation, by means of the secondary shaft (54), said upper part (52A) of said primary shaft (52) to said motorized means (80) so as to drive in rotation said upper part (30A) for transfer of the containers (12), and
- a disengaged state when said at least one upper part (30A) for transfer of the containers (12) from the wheel (30) is in the transfer position to disconnect said secondary shaft (54) from said motorized means (80).
